# EUROPÄISCHE PATENTSCHRIFT

(11) **EP 2 548 617 B1**
(45) Veröffentlichungstag und Bekanntmachung des Hinweises auf die Patenterteilung: **20.08.2014**
(21) Anmeldenummer: 12177266.9
(22) Anmeldetag: 20.07.2012
(51) Int. Cl.: A61B 18/20, A61N 5/06

(54) **Lasertherapiesystem mit UVA- und Laser-Licht zur gerichteten Erzeugung einer dermalen Kollagen-Matrix**
Laser therapy system with UVA and laser light for directed generation of a dermal collagen matrix
Système de traitement laser avec lumière UVA et laser destinée à générer de façon orientée une matrice de collagène dermique

(30) Priorität: 20.07.2011 DE 102011052002
(43) Veröffentlichungstag der Anmeldung: 23.01.2013
(73) Patentinhaber: Telesto GmbH, 89081 Ulm (DE)
(72) Erfinder: Depfenhart, Markus, Dr., 22417 Hamburg (DE); Müller, Jörg, Prof., 21244 Buchholz (DE)
(74) Vertreter: Farago, Peter Andreas

(56) Entgegenhaltungen:
- WO-A2-2008/089344
- US-A1- 2007 106 284
- US-A1- 2009 069 741

## Beschreibung

### Erfindungsgebiet

Die vorliegende Erfindung betrifft ein Lasertherapiesystem, das im Wesentlichen eine erste Lichtquelle im UVA-Bereich, eine zweite Laser-Lichtquelle mit einem zweiten Wellenlängenbereich, eine Optik und eine Steuer- und Navigationseinheit umfasst, mit der fokussierte Lichtstrahlbündel als Lichtspots exakt in einer Haut bzw. in einem Hautgewebe positioniert und appliziert werden können. Dadurch wird eine physiologische und anatomisch exakte Remodellierung des Hautskelettes ermöglicht.

### Hintergrund der Erfindung

Derzeit ist eine Vielzahl von Verfahren und Lasertherapiesystemen für eine Hautverjüngung und zur Narbenbehandlung bekannt und verfügbar, die beispielsweise auf fraktionierter Laserablation oder mesotherapeutischen Kollageninduktionsverfahren beruhen. Für die fraktionierte Laserablation sind beispielsweise Geräte wie Fraxel ® bekannt und im Einsatz. Eine Denaturierung von Kollagen und damit ein "Shrinking" des Gewebes kann auch durch Radiofrequenz, wie beispielsweise durch ein Thermage ®-Gerät erzielt werden. Solche Verfahren und Geräte basieren meist darauf, dass entweder mittels eines mechanischen oder eines thermischen Therapiegerätes, wie z.B. ein Laser, Verletzungen in der Haut, je nach Gerät in unterschiedlicher Form und Tiefe, erzeugt werden. Daran anschließende körpereigene Reparaturvorgänge führen dann durch Abbau des destruierten Gewebes und Wiederaufbau des Gewebes letztlich zu der Hautverjüngung oder einfach nur zu einer epidermalen und/oder subepidermalen Narbe, die auch eine Straffung der Haut bewirken kann. Abzugrenzen hiervon sind sog. mesotherapeutische Verfahren unter denen man auch im Entferntesten eine perkutane Kollageninduktionstherapie oder kurz "Needling" subsumieren kann. Hierbei werden beispielsweise mittels eines Nadelrollers Mikroblutungen im Stratum Papillare der Haut erzeugt, wodurch es in der Dermis zu einer Freisetzung von korpuskulären Blutbestandteilen wie hauptsächlich Thrombozyten kommt. Unter anderen aus den Thrombozyten werden dann auch Wachstumsfaktoren, wie beispielsweise TGF β3, VEGF, EGF, freigesetzt. Diese fördern den Ab- und Umbau von Narben und die Bildung von gerichtetem Kollagen in der Dermis, wodurch die Haut bis zu einem gewissen Grad regeneriert bzw. verjüngt wird. Bei diesem Behandlungsverfahren findet auch eine perioperative Behandlung mit Vitamin A- und Vitamin C-haltigen Cremes statt, um durch die topische Applikation hohe lokale Level an Vitamin A und Vitamin C, die wichtige Cofaktoren bzw. Coenzyme für die Bildung von Kollagen und Elastin sind, in einer Zielregion der Haut für die Bildung von Kollagen und Elastin zu erreichen.

All den, zuvor genannten Verfahren und Geräten ist gemein, dass sie entweder hauptsächlich oberflächenwirksam oder relativ invasiv und daher mit hohen Risiken und Nebenwirkungen behaftet sind, bei einem befriedigenden makroskopischen Ergebnis, aber keinesfalls bei einem anatomisch rekonstruierenden Effekt. Bei dem "Needling" ist abgesehen von der Schmerzhaftigkeit der Behandlung die Infektionsgefahr, sowohl für den Therapeuten bzw. Behandler, als auch für den Patienten nicht zu unterschätzen. So ist ein kosmetisch-ästhetisches Verfahren, bei dem es zu Blutaustritt an einer Oberfläche der Haut kommt im Zeitalter von HIV und Hepatitis nicht unkritisch zu sehen. Ferner bietet ein solches Verfahren wenig Möglichkeit zu einer Standardisierung und damit zu einer Qualitätssicherung, da Parameter wie Anpressdruck des Nadelinstrumentes und damit eine Tiefe einer Nadelapplikation in der Haut, ein Richtungswechsel, der eine Gitterwirkung hat, ein Rasterschub und damit evtl. eine mehrfache Perforation des selben Hautareals individuell vom Therapeuten bzw. Behandler abhängig und daher nicht messbar oder vergleichbar sind.

WO 2008/089344 A2 (Neev) beschreibt eine Vorrichtung und ein Verfahren einer IR-Laser Applikation für eine dermale Laserapplikation, wobei hierbei mehrere fokussierte Lichtstrahlbündel mit bis zu 10.000 Fokuspunkten bzw. Lichtspots erzeugt und appliziert werden können. Indem die Lichtstrahlbündel mit einer bestimmten Brennweite fokussiert werden, tritt das jeweilige Lichtstrahlbündel mit einer ersten Querschnittsfläche und mit einer ersten Leuchtdichte in die Haut ein und erzeugt im jeweiligen Fokuspunkt im Behandlungsbereich in der Haut eine zweite, viel höhere Leuchtdichte als die erste Leuchtdichte, die im Fokuspunkt groß genug ist, um eine lichtinduzierte Reaktion in der Haut hervorzurufen. Beschriebene Anwendungen umfassen beispielsweise eine Anregung oder Abtötung von Haarwurzeln, Behandlung von Akne, Tattoos, Farbänderungen, Bräunung, Augenbehandlung inklusive einer Erzeugung von subcutanen Kavitäten. Bei der Erzeugung von subcutanen Kavitäten kommt es zu Ab-/und Umbauvorgängen in der Haut und zur Bildung von Kollagenfasern. Eine Brennweite und eine Energie des jeweiligen Lichtstrahlbündels sind dabei so gewählt, dass die Oberhaut nicht perforiert wird. Ein Einsatz einer optischen Kohärenztomographie (OCT) als bildgebendes Verfahren ermöglicht eine Betrachtung eines Aufbaus der Haut beispielsweise mit Haarwurzeln, Blutgefäßen, Matrix Zellen und Papillen, auch in perspektivischer Ansicht, so dass dieser Bereich gezielt mit den Lichtspots beschossen werden kann. Die Vorrichtung kann auch ein Kühlelement für die Haut umfassen, das jedoch kaum notwendig ist.

WO 2008/001 284 A2 (Verhagen et al.) beschreibt ein Lasertherapiesystem und ein Verfahren für eine Hautbehandlung in der Tiefe der Haut, das auf Laser induced Breakdown (LIOB) basiert. Mit einer Kamera und einem Monitor können Falten sichtbar gemacht werden, um darunter in der Tiefe der Haut gezielt mit einem Laser zu behandeln.

WO 02/053 050 A1 (Altshuler et al.) beschreibt eine Vorrichtung und ein Verfahren für eine Licht-induzierte Behandlung in der Tiefe der Haut, wobei die Lichtquelle entweder eine Laser-Lichtquelle oder auch eine nicht kohärente Lichtquelle sein kann. Dabei werden mehrere fokussierte Lichtstrahlbündel gleichzeitig erzeugt, indem die Optik ein Linsensystem mit vielen, netzartig angeordneten Linsen umfasst, die integral oder nicht integral ausgeführt sind. Ferner umfasst die Vorrichtung ein Kühlelement für die Haut, das entweder eine Hautkontaktplatte oder das Linsensystem selbst sein kann.

Aus US 7 198 634 B2 ist ein Lasertherapiesystem zur Hautbehandlung bekannt, das sowohl eine Infrarotlichtquelle als auch eine Lichtquelle mit violettem oder blauem Spektrum aufweist.

US 2009/0069741 A1 lehrt ein System, welches keine zweite Lichtquelle aufweist.

Bei allen derzeitigen, laserbasierten oder anderweitigen Verfahren wird eine anatomisch physiologische Rekonfiguration bzw. Remodellierung des Gewebes der Haut nicht berücksichtigt.

### Zusammenfassung der Erfindung

Daher besteht eine Aufgabe der Erfindung in der Bereitstellung eines Lasertherapiesystems, das die Nachteile aus dem Stand der Technik beseitigt und eine anatomisch physiologische Rekonfiguration des Gewebes der Haut und dabei insbesondere der Kollagen- und Elastinmatrix der Haut ermöglicht.

Eine weitere Aufgabe der Erfindung besteht darin, mit dem Lasertherapiesystem eine über einem Behandlungsbereich der Haut liegende Oberhaut bzw. Epidermis nicht zu perforieren und möglichst wenig zu schädigen.

Die vorstehenden Aufgaben werden von einem Lasertherapiesystem zur Hautbehandlung in einem volumenartigen Behandlungsbereich in einer Tiefe der Haut gemäß den Merkmalen der unabhängigen Ansprüche 1 und 15 gelöst.

Weitere vorteilhafte Ausbildungen der Erfindung sind in den abhängigen Ansprüchen angegeben.

Mit dem der Erfindung zugrunde liegenden Lasertherapiesystem kann die Bildung von Kollagen und Elastin in der Haut entsprechend ihrer physiologischen Textur mikroskopisch präzise zunächst in einem ersten Schritt als eine Kollagen- und Elastin-Grundstruktur und danach in einem zweiten Schritt als weiter sich anlagerndes Kollagen induziert werden. Dadurch ist es möglich, das "Hautskelett" nicht nur zu induzieren, sondern physiologisch und narbenfrei und ohne Perforation der Oberhaut zu regenerieren.

Außerdem können das Verfahren und das Lasertherapiesystem auch zum narbenfreien Verschluss von Wunden eingesetzt werden.

Darüber hinaus ist es auch vorstellbar, das Verfahren und das Lasertherapiesystem außer für die Haut auch für andere Gewebe einzusetzen, um diese beispielsweise zu verbinden.

Eine bevorzugte Ausführungsform gemäß der vorliegenden Erfindung ist in nachfolgenden Zeichnungen und in einer detaillierten Beschreibung dargestellt, soll aber die vorliegende Erfindung nicht darauf begrenzen.

### Kurzbeschreibung der Zeichnungen

Fig. 1 zeigt ein Lasertherapiesystem als Blockdiagramm mit verschiedenen Komponenten, wobei ein Austritt eines Lichtstrahlbündels aus bzw. hinter einer Adapterplatte erfolgt, die auf einem Bereich einer Haut, von der die Epidermis und die Dermis skizziert sind, aufgelegt ist. In der unteren Hälfte des Bildes sind ein Monitor, der Bilddaten von einem Kamerasystem zur Darstellung des Hautbereichs empfängt, ein Navigationssteuergerät und eine separate Abgabeeinheit zur Abgabe eines Mittels skizziert.
Fig. 2 zeigt ein Lasertherapiesystem als Blockdiagramm mit den Komponenten wie in Bild 1 dargestellt, wobei hinter der Adapterplatte gleichzeitig mehrere Lichtstrahlbündel austreten.

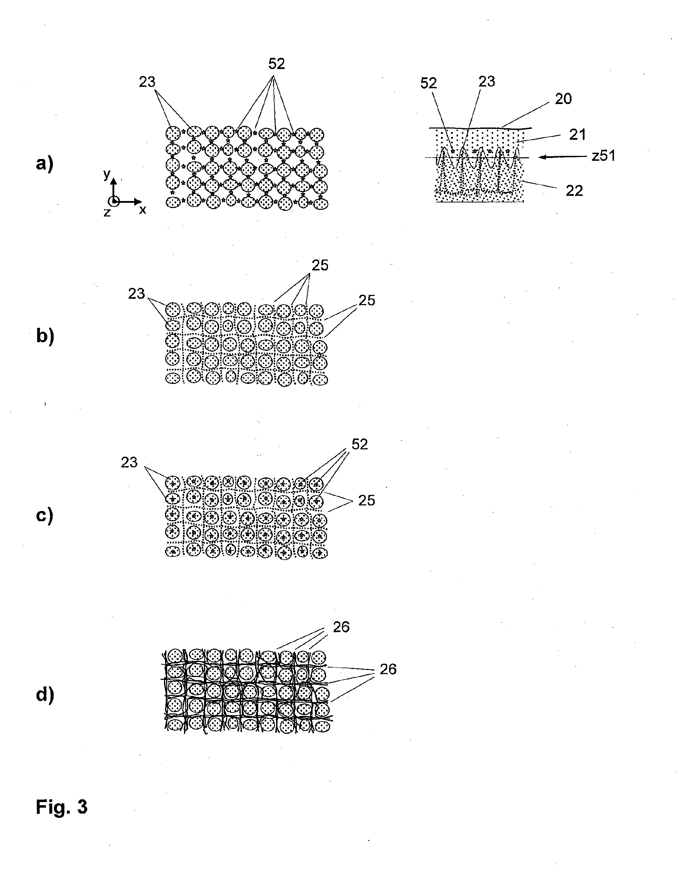

Fig. 3a zeigt links im Bild in Draufsicht ein schematisiertes Schnittbild einer in einer Tiefe der Haut liegenden papillären Dermis, die in einer x/y-Ebene liegend parallel zur Hautoberfläche liegt, wobei im Wesentlichen die Ränder der Papillen in der Schnittebene dargestellt sind. Die zwischen den Rändern der Papillen dargestellten Sterne zeigen applizierte Lichtspots im UVA-Wellenlängenbereich an. Rechts im Bild ist ein schematisiertes vertikales Schnittbild der Haut dargestellt, das Aufschluss über eine Höhenverteilung und -beschaffenheit der Papillen mit Blutgefäßen gibt. Die darin skizzierten Sterne stellen Lichtspots dar.
Fig. 3b zeigt in Draufsicht das schematisierte Schnittbild desselben Hautbereichs wie in Fig. 3a dargestellt, jedoch mit einer gitterförmigen Kollagen- und Elastin-Grundstruktur, die als Folge einer Bestrahlung mit den UVA-Lichtspots als ein Crosslinking erzeugt worden sind, wobei der Hautbereich zuvor mit dem Mittel behandelt worden ist.
Fig. 3c zeigt in Draufsicht das schematisierte Schnittbild desselben Hautbereichs wie in Fig. 3b dargestellt, jedoch mit sternförmiger Darstellung von zweiten Laser Lichtspots, die auf den Papillenspitzen positioniert sind.
Fig. 3d zeigt in Draufsicht das schematisierte Schnittbild desselben Hautbereichs wie in Fig. 3c dargestellt, jedoch mit weiterem, durch Fibroblasten entlang der richtungsgebenden Kollagen- und Elastin-Grundstruktur aufgebautem Kollagen.
Fig. 4a zeigt in einer Seitenansicht schematisch eine Lichtquelleneinheit, die verschiedene diskrete Lichtquellen aufweist, ein erstes Linsensystem zur Lichtstrahlaufweitung, ein zweites Linsensystem zur Lichtstrahlfokussierung und eine Adapterplatte mit einem jeweiligen Lichtstrahlverlauf.
Fig. 4b zeigt in Draufsicht von oben schematisch das zweite Linsensystem der Figur 4a mit darin enthaltenen 16 Linsen zur Lichtstrahlfokussierung, wobei die dünnen Linien in den Linsen die Strahldurchmesser beim Durchgang durch die Linsen und die Sterne in deren Zentren die fokussierten Lichtspots darstellen.
Fig. 4c zeigt in Draufsicht ein weiteres, größeres als in Fig. 3a skizziertes, schematisiertes Schnittbild der papillären Dermis der Haut. Darüber bzw. darin liegend ist eine Matrix nebeneinander angeordneter Fokuspunkte (X1/Y1) bis (X5/Y4) als Sterne dargestellt, die in ihrer Lage Hardware bedingt sind, und die jeweils als Lichtspot von der Steuereinheit angesteuert werden können.
Fig. 5 zeigt in einer Seitenansicht schematisch die Lichtquelleneinheit, das erste Linsensystem, einen Lichtleiter mit Lichtfaserbündeln, einen Lichtadapterkopf mit dem zweiten Linsensystem und der Adapterplatte.
Fig. 6a zeigt ein Diagramm mit Absorptionskennlinien von oxygeniertem und nicht-oxygeniertem Hämoglobin in Abhängigkeit von der Lichtwellenlänge. Auf der Ordinate ist der spezifische Absorptionskoeffizient aufgetragen. Quelle: J.Eichmeier: Medizinische Elektronik, 2. Auflage, 1991, Springer Verlag.
Fig. 6b zeigt einen schematischen Aufbau und eine Anordnung eines Kamerasystems, das über die Adapterplatte an die Haut angekoppelt ist, wobei die Haut als ein vertikaler Schnitt in die Haut schematisch dargestellt ist.
Fig. 6c zeigt schematisch in einer Draufsicht ein Detektionsfeld und ein darum herum angeordnetes Beleuchtungsfeld des Kamerasystems.

### Detaillierte Beschreibung einer bevorzugten Ausführungsform

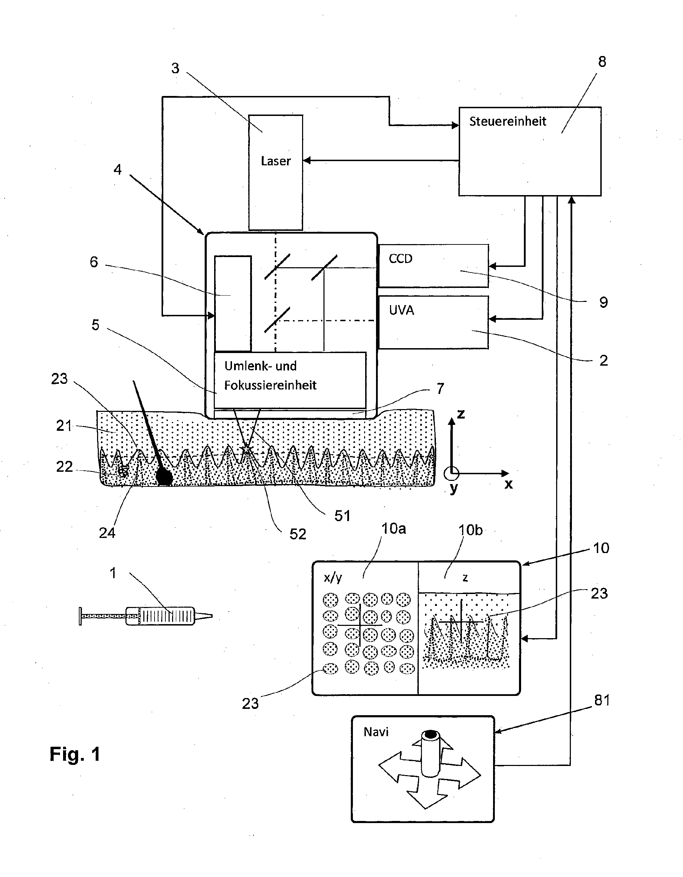

Fig. 1 zeigt eine schematische Darstellung eines erfindungsgemäßen, bevorzugten Lasertherapiesystems, umfassend eine separate Abgabeeinheit 1 zur Abgabe eines Photosensitizer-haltigen Mittels, eine erste Lichtquelle als eine steuerbare UVA-Lichtquelle 2 mit einer ersten Lichtwellenlänge im UVA Bereich, eine zweite Lichtquelle 3 als eine steuerbare Laser-Lichtquelle mit einer zweiten Lichtwellenlänge und ein optisches System 4, an das die erste 2 und die zweite Lichtquelle 3 angeschlossen sind, so dass deren Lichtstrahlen, auch Lichtstrahlbündel genannt, durch das optische System 4 aufgenommen und zu einer Adapterplatte 7 am Ausgang des optischen Systems 4 weitergeleitet werden und aus dieser hervortreten. So tritt das jeweilige UVA-Licht der ersten Lichtquelle und/oder das zweite Laser-Licht der zweiten Lichtquelle 3 aus dem Lasertherapiesystem hinter der Adapterplatte 7 bevorzugt als ein fokussiertes Lichtstrahlbündel 51 aus und weist hinter der Adapterplatte 7 in einem Fokuspunkt 52 einen Lichtspot auf. Dabei hat das fokussierte Lichtstrahlbündel 51 für die erste und die zweite Lichtwellenlänge bevorzugt eine gleiche Brennweite und eine gleiche Position des Fokuspunkts 52 in einem x/y/z-Koordinatensystem. Die Adapterplatte 7 ist für die verwendeten Lichtwellenlängen aus einem hochtransparenten Material und bevorzugt aus Saphirglas beschaffen, das zudem bevorzugt gut hautverträglich ist. Eine zusätzliche Hautadapterplatte oder -folie zwischen der Adapterplatte 7 und der Haut eines Patienten ist ebenso vorstellbar. Bevorzugt sind die Adapterplatte 7 sowie das gesamte Lasertherapiesystem für eine Anwendung zu einer Hautbehandlung von Patienten ausgelegt. In Fig. 1 ist die Adapterplatte 7 in einer Seitenansicht auf einer Haut liegend skizziert, wobei die Haut als vertikales Schnittbild in einer x/z-Ebene dargestellt ist. Ein Behandlungsbereich der Haut unter der Adapterplatte 7 besteht im Wesentlichen aus einer Oberhaut, einer Epidermis 21 und einer Dermis 22 mit darin liegenden, hügeligen Papillen 23 und gepunktet skizzierten Blutgefäßen 24.

Die Lichtstrahlen der UVA-Lichtquelle 2 und der zweiten Lichtquelle 3 werden im optischen System 4 bevorzugt, wie skizziert, mittels halbdurchlässiger Spiegel zu einem Lichtstrahlbündel gebündelt und dann einer Lichtstrahlumlenk- und Fokussieroptik 5 zugeführt. Die Lichtstrahlumlenk- und Fokussieroptik 5 besteht bevorzugt aus einer Vielzahl an Linsen und anderen Optikelementen, wie Spiegel, Prismen, Lichtleiter und Kombinationen daraus, die zu einer Lichtstrahlaufweitung und -Fokussierung, für eine Lichtstrahlumlenkung bzw. die für eine gesteuerte definierte Navigation des fokussierten Lichtspots hinter der Adapterplatte 7 nötig sind. Die im optischen System 4 skizzierten Spiegel und halbdurchlässigen Spiegel können auch als Teil der Lichtstrahlumlenk- und Fokussieroptik 5 aufgefasst werden. Einige Optikelemente, die für die Lichtstrahlumlenkung verantwortlich sind, sind bevorzugt mit Aktuatoren einer elektromechanischen Verstellvorrichtung 6 verbunden, so dass über diese die Lichtstrahlen elektrisch gesteuert umgeleitet werden können, um den entsprechenden, resultierenden Lichtspot bzw. den Fokuspunkt 52 zu navigieren. Die Aktuatoren sind dabei beispielsweise Motoren, Schrittmotoren, piezoelektrische Stellantriebe oder Magnetstellantriebe in Verbindung mit Hebelübersetzungen, Getriebeteilen und Kombinationen daraus. Es ist auch vorstellbar, dass die halbdurchlässigen Spiegel durch Aktuatoren bewegt werden können. Rückmeldesignale von den Aktuatoren und Stellwinkelsensorsignale werden bevorzugt auch verarbeitet, um die Lichtstrahlumlenkung exakt zu halten.

Eine Steuereinheit 8 steuert sowohl die Aktuatoren der Verstellvorrichtung 6 als auch die UVA-Lichtquelle 2 und die zweite Lichtquelle 3, deren Lichtintensitäten mit einstellbaren Puls-Pause-Zeiten und Repetitionsraten steuerbar sind, was bedeutet, dass die jeweilige Lichtquelle 2 bzw. 3 dadurch definierte Lichtenergien abgibt. Ein Lichtmeßsignal zu Rückkopplungs- und Regelzwecken wird bevorzugt auch verwendet.

Als UVA-Lichtquelle 2 wird bevorzugt ein pulsbarer Laser eingesetzt, es ist aber auch denkbar, eine nichtkohärente UVA-Lichtquelle 2 mit einem breiteren Lichtstrahl, als es bei einem Laserstrahl der Fall ist, zu verwenden, der durch eine Blende und einen Shutter entsprechend einstellbar und pulsbar gemacht werden kann. Die erste Lichtwellenlänge der UVA-Lichtquelle 2 und das Photosensitizer-haltige Mittel, sind bevorzugt aufeinander abgestimmt, indem die erste Lichtwellenlänge von dem Photosensitizer-haltigen Mittel genügend gut absorbiert wird, so dass applizierte erste Lichtspots im UVA Bereich, auch vereinfacht UVA-Lichtspots genannt, ein Kollagen- und Elastin-Crosslinking von Eckpunkt zu Eckpunkt der Papillen 23 induzieren. Hierbei ist zu erwähnen, dass immer nur eine bestimmte Kopf- /und Schwanzregion im Sinne der physiologischen Lysyloxidasereaktion verbunden werden. Eine solche Verbindung wird durch ein Photosensitizer-haltiges Mittel, wie beispielsweise Riboflavin, vermittelt. Das Photosensitizer-haltige Mittel wird in einer speziellen pharmakologischen Formulierung auf die Haut aufgebracht, die die Epidermis 21 gut penetrieren kann und somit die papilläre Dermis 22 als ihre Zielstruktur erreicht. Durch das Photosensitizer-haltige Mittel kann die übertragene Energie des UVA- Lasers gering gehalten werden und Kollateralschäden durch UVA - Strahlung, die zytotoxisch ist, vermieden werden. Ferner kann die Zielregion damit definiert werden. Es wird im Übrigen pro Lichtspot nur so viel Energie mit dem UVA- Laser appliziert, wie auch von dem Photosensitizer-haltige Mittel in diesem Bereich absorbiert werden kann. Denkbar ist auch die Vermengung des Photosensitizer-haltige Mittels mit einer fluoreszierenden Substanz, wodurch mit einer Kamera mit einem der Fluoreszenzwellenlänge entsprechender speziellen Farbfilter und/oder Beleuchtungsquelle die Eindringtiefe des Photosensitizer-haltige Mittels in die Zielregion kontrolliert werden kann.

Das UVA-Licht weist die erste Lichtwellenlänge von bevorzugt 340 - 450 nm auf. Das oder die fokussierten UVA-Lichtstrahlbündel, die hinter der Adapterplatte 7 in die Haut eintreten, weisen bevorzugt eine erste Lichtintensität auf, die 100µW - 100mW pro fokussierten Lichtstrahlbündel 51 entsprechen. Im Fokuspunkt 52 ist die Lichtintensität um den Anteil, der zuvor in der Haut absorbiert wurde, kleiner. Der Fokuspunkt weist bevorzugt einen Durchmesser von 5 - 10 µm auf, kann aber bevorzugt auch bis 100 µm eingestellt werden. Die Fokustiefe hinter der Adapterplatte 7 in der Haut ist bevorzugt im Behandlungsbereich mit einer Tiefe von 0,03-3 mm einstellbar bzw. steuerbar. Die Fokustiefe ist bevorzugt auch um eine Dicke einer eventuell zwischen der Adapterplatte 7 und der Haut eingebrachten Glasplatte oder Folie erweiterbar. Die Pulsdauern betragen bevorzugt 1 ms - 1 s, können aber bevorzugt auch kürzer und länger eingestellt werden. Bei der UVA-Lichtbestrahlung ist es auch vorstellbar, die Lichtstrahlenbündel 51 in Sonderfällen nicht fokussiert zu erzeugen, sondern sie planar im Behandlungsbereich auf der Haut zu applizieren.

Als die zweite Lichtquelle 3 wird bevorzugt ein pulsbarer Laser eingesetzt. Dabei ist es vorstellbar, den Laserstrahl aufzuweiten und durch eine Blende und durch einen Shutter zu leiten, um dadurch die Lichtleistung zusätzlich zu regulieren oder zu kontrollieren. Die zweite Lichtwellenlänge, eine zweite Lichtintensität, eine zweite Pulsdauer und eine zweite Pulsfrequenz bzw. Repetitionsrate der zweiten Lichtquelle 3 sind bevorzugt auf eine gute Absorption des Lichts und auf eine Energiefreisetzung im Fokuspunkt 52 abgestimmt, um die Endarteriolen möglichst gezielt und ohne größere Kollateralschäden für korpuskulare Blutbestandteile permeabel zu machen. Bevorzugt werden die Laserparameter so eingestellt bzw. angesteuert, dass sich im Fokuspunkt 52 mit dem Gewebe entweder eine thermische, eine photoablative oder eine plasmainduzierte Wechselwirkung ergibt. Dabei weist das zweite Laser--Licht die zweite Lichtwellenlänge von bevorzugt 450-550nm oder besonders bevorzugt von 550-650nm oder von 650-800nm oder von 800-2000nm im IR Bereich auf. Die zweite Lichtwellenlänge wird dabei bevorzugt so gewählt und eingestellt, dass die zweite Lichtwellenlänge besonders gut von Hämoglobin und möglichst wenig vom restlichen Gewebe der Haut absorbiert wird. Das fokussierte zweite Laser-Lichtstrahlbündel, das hinter der Adapterplatte 7 in die Haut eintritt, weist bevorzugt die erste Querschnittsfläche von 1 - 30 mm² auf, die aber auch größer ausfallen kann. Die zweite Lichtintensität entspricht bevorzugt 1 mW - 100 W pro fokussiertem Lichtstrahlbündel 51. Im Fokuspunkt 52 bzw. im Lichtspot ist die Lichtintensität um den Anteil, der zuvor in der Haut absorbiert wurde, kleiner. Der Fokuspunkt weist bevorzugt einen Durchmesser von 10 - 100 µm auf. Die Fokustiefe hinter der Adapterplatte 7 in der Haut liegt bevorzugt in einem Bereich von 0,03-3 mm oder größer und ist einstellbar bzw. steuerbar. Die Pulsdauern betragen bevorzugt 10 ps -100 µs, können aber auch kürzer und länger eingestellt werden.

Bevorzugt ist an die Steuereinheit 8 ein Navigationssteuergerät 81, beispielsweise als Joystick mit entsprechenden Steuerknöpfen oder als Track-Ball ausgebildet, angeschlossen. Damit lässt sich bevorzugt die Position des Fokuspunktes 52 im Behandlungsbereich der Haut in x-, y- und z-Richtung genau positionieren. Andere und/oder zusätzliche Eingabe- bzw. Steuermöglichkeiten, beispielsweise per Touchscreen-Monitor, per Spracherkennung oder per Bilderkennung von Gestiken oder Mimiken, sind ebenso denkbar.

Bevorzugt umfasst das Lasertherapiesystem auch ein Kamerasystem 9 und einen Monitor 10, über die sich ein behandelnder Arzt ein Bild über die zu behandelnde Haut und über die jeweiligen Lichtspot Positionen für das UVA- und das zweite Laser Licht im Behandlungsbereich machen kann, wo die jeweiligen Lichtspots erzeugt bzw. wohin sie navigiert werden sollen. In Fig. 1 ist beispielsweise auf dem Monitor 10 links im Bild eine erste Darstellung 10a skizziert, die den Behandlungsbereich als horizontales Schnittbild in einer x/y Ebene, Ränder der Papillen 23 und ein Fadenkreuz, das die Position eines zu erzeugenden Lichtspots angibt, gezeigt. Rechts im Bild des Monitors 10 wird gleichzeitig eine zweite Darstellung 10b des vertikalen Schnitts in einer x/z-Ebene des Behandlungsbereichs der Haut angezeigt, wobei das Fadenkreuz die x/z-Position des zu erzeugenden Lichtspots anzeigt. Eine dreidimensionale Darstellung des Behandlungsbereichs oder einer Vergrößerung davon ist ebenso vorstellbar und bevorzugt vom behandelnden Arzt auswählbar.

Das Kamerasystem 9 wird bevorzugt im gleichen Strahlengang wie das UVA- und das zweite Laser-Licht, das durch die Adapterplatte 7 geführt wird, gehalten. Dabei wird bevorzugt ein OCT basiertes bildgebendes System eingesetzt, mit dem sich der Behandlungsbereich der Haut dreidimensional auch in der Tiefe darstellen läßt. Dabei wird bevorzugt Licht einer Wellenlänge zwischen 340nm und 550nm verwendet. Es ist bei der Bildaufnahme und -Verarbeitung aber auch vorstellbar, dass, ähnlich wie bei der digitalen Subtraktionsangiographie, zwei Kamerabilder mit unterschiedlichen Parametern gemacht werden, die dann zusammen so verarbeitet werden, dass sich eine gewünschte Gewebe- oder Gefäßstruktur kontrastieren läßt. Im Fall des vorliegenden Kamerasystems 9 werden bevorzugt unterschiedliche Lichtwellenlängen zur Beleuchtung der Haut verwendet, wobei ein erstes Kamerabild bei einer ersten und ein zweites Kamerabild bei einer zweiten Beleuchtungswellenlänge gemacht werden. Anschließend werden das erste und das zweite Kamerabild so verarbeitet, dass sämtliche Strukturen des Hautbehandlungsbereichs weitgehend wegsubtrahiert werden und im Wesentlichen nur noch die Papillen 23 des Stratum Papillare und die Blutgefäße sichtbar bleiben. Vorzugsweise lassen sich damit auch Kollagen- und Elastin-Fasern hervorheben, was auswählbar ist. Bevorzugt wird dabei die UVA-Lichtquelle 2 als eine der Beleuchtungslichtquellen verwendet. Vorzugsweise werden die erzeugten Lichtspots als kleine Lichtpunkte auf dem Monitor 10 dargestellt. Vorzugsweise werden auch die schon behandelten Stellen am Monitor markiert, damit der Arzt einfach erkennen kann, wo noch zu behandeln ist; dies gilt für die Behandlung im UVA-Wellenlängenbereich, wie auch im zweiten Laser-Licht-Wellenlängenbereich. Alternativ zu dem OCT basierenden System kann auch ein auf einer Fluoreszenzmikroskopie oder einer Fluoreszenztomographie basierendes bildgebendes System eingesetzt werden, bei dem entweder mit injiziertem oder mit topisch appliziertem Fluoreszenzfarbstoff und einer Anregung durch Licht oder Laser eine Eigenfluoreszenz erzeugt wird und auswertbar ist. Nachfolgende bildverarbeitende Auswertungen können dabei die gleichen sein wie oben beschrieben.

Bevorzugt wird das bildgebende System, umfassend das Kamerasystem 9, den Monitor 10 und eine Mikroprozessor-Einheit, die auch Teil des Steuersystems sein kann, auch dazu verwendet, eine Mustererkennung durchzuführen. Die Mustererkennung ist so ausgelegt, dass bevorzugt der Bereich der papillären Dermis 22 des Behandlungsbereichs erkannt wird, so dass die Papillen 23 und der dazwischen liegende Bereich, wo die UVA-Lichtspots zu setzen sind, erkannt werden. Die x/y/z-Koordinaten der UVA-Lichtspots ergeben zusammen eine UVA-Lichtspot-Matrix als eine erste Lichtspot-Matrix. Durch die Erkennung der Papillen 23 und der darin liegenden Endarteriolen erzeugt die Mustererkennung bevorzugt auch automatisiert die x/y/z-Koordinatenpunkte, wo zweite Laser-Lichtspots des zweiten Laser-Lichts zu setzen sind. Die x/y/z-Koordinaten der zweiten Lichtspots ergeben zusammen eine zweite Lichtspot-Matrix. Bevorzugt wird dann eine automatische oder semiautomatische Erzeugung der UVA- oder der zweiten Laser-Lichtspots durch die Steuereinheit 8 vorgenommen. Bei der semiautomatischen Lichtspot-Erzeugung wird bevorzugt ein Lichtspot nach dem anderen am Monitor 10 markiert und vom Arzt freigegeben, oder es werden sequentielle Abfolgen mit einer bestimmten Anzahl von Lichtspots auf dem Monitor 10 markiert, vom Arzt freigegeben und dann automatisch ausgeführt. Eine bevorzugte, weitere Mustererkennung erkennt ein Verschieben der Adapterplatte 7 auf der Haut und transformiert die x/y/z-Koordinaten der örtlich schon bestimmten, zu erzeugenden Lichtspots auf den aktuellen Behandlungsbereich unter der Adapterplatte 7, so dass die Lichtspots noch an gleichen Stellen, wie vorher bestimmt, erzeugt werden. Bevorzugt werden die behandelten und die noch nicht behandelten Bereiche des Stratum Papillares erkannt, so dass bei der semiautomatischen oder automatischen Lichtspot-Erzeugung bevorzugt nur die noch nicht behandelten Bereiche bestrahlt werden. Die Mustererkennung erkennt bevorzugt auch automatisch die richtige Tiefe z der Zielregion, um den bzw. die Fokuspunkte 52 auf die richtige Tiefe z durch die Steuereinheit 8 einzustellen oder voreinzustellen, so dass der Arzt in diesem Fall nur noch bestätigen muss.

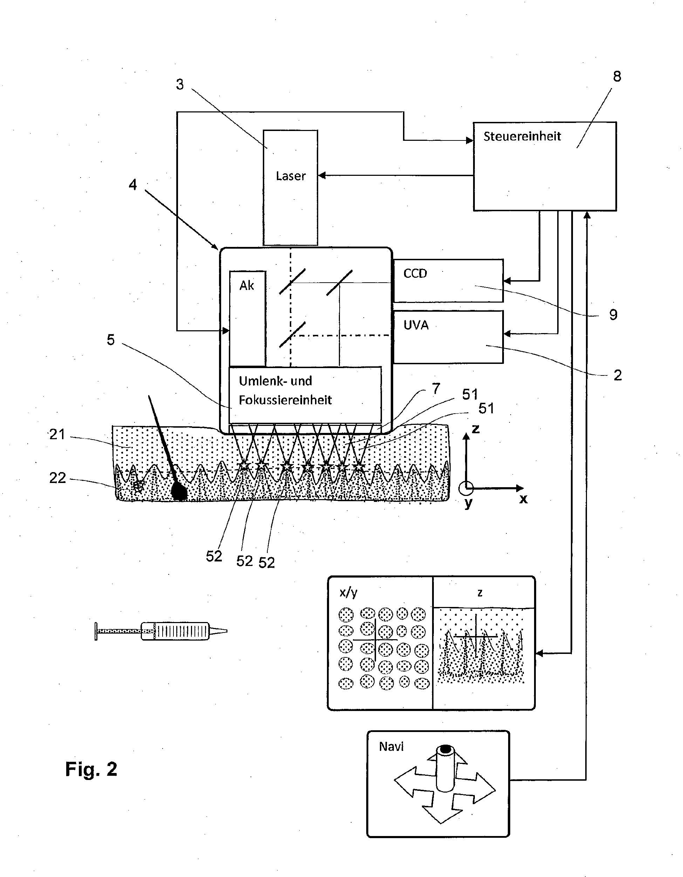

Fig. 2 zeigt schematisch einen ähnlichen Aufbau des Lasertherapiesystems, wie in Fig. 1 skizziert, nur dass diese Ausführungsform gleichzeitig oder automatisch/semiautomatisch sequentiell mehrere fokussierte Lichtspots an den Fokuspunkten 52, die als Sterne skizziert sind, zu erzeugen vermag. Unter semiautomatisch wird hier verstanden, dass nachdem zunächst die x/y/z-Koordinaten aller Lichtspots in dem Behandlungsbereich der Haut unter der Adapterplatte 7 durch die bevorzugte Mustererkennung als die jeweilige Lichtspot-Matrix bestimmt worden sind und der Arzt diese zur Lichtspot-Applikation freigegeben hat, dann eine schnelle Abfolge einer oder mehrerer gleichzeitiger Lichtspot-Salven oder einer sequentielle Abfolge der Lichtspots durch die Steuereinheit 8 vorgenommen wird. Dies gilt jeweils für die Lichtspot-Matrix im UVA-Bereich und der zweiten Wellenlänge. Dafür sind das optische System 4, die Lichtstrahlumlenk- und Fokussieroptik 5 und die Aktuatoren der Verstellvorrichtung 6 bevorzugt so ausgelegt, dass beispielsweise schnelle Piezo-Aktuatoren und optische Elemente mit möglichst geringer Masse eingesetzt werden, um möglichst schnelle, präzise und geräuschlose Verstellungen zu ermöglichen. Dazu ist es auch denkbar, im Lasertherapiesystem mechanische, gegenläufige Bewegungen von Massen auszuführen, um Vibrationen, Geräusche und Verschleiß zu reduzieren und dadurch auch die Präzision zu erhöhen. Auch werden die Verstellbewegungen bevorzugt nicht durch abrupte, sondern durch stetig ansteigende bzw. abfallende Verstellbeschleunigungen angesteuert, um dadurch optimal schnelle Einschwingzeiten der Lichtstrahlumlenk- und Fokussieroptik 5 zu erzielen.

Bevorzugt werden die Lichtspots der jeweiligen Lichtspot-Matrix, die, nochmals zur Klarheit, räumlich keine äquidistanten, sondern physiologisch bedingte Abstände zueinander aufweisen, entweder zunächst in nur einer Tiefe z des Behandlungsbereichs der Haut in einer ersten gleichzeitigen Salve oder in einer ersten sequentiellen Abfolge erzeugt, so dass für eine nächste Behandlungsebene bzw. Tiefe z durch den Arzt eine nächste Salve oder nächst Abfolge an Lichtspots freigegeben werden müsste. Oder es werden alle Lichtspots der volumenartigen jeweiligen Lichtspot-Matrix auf einmal semiautomatisch sequentiell oder gleichzeitig erzeugt. Dabei kann beispielsweise auch eine maximale Anzahl von applizierten Lichtspots nach einer Freigabe durch den Arzt eingestellt werden. Dabei werden bevorzugt im jeweiligen Lichtwellenlängenbereich die noch nicht behandelten Bereiche von den schon mit Lichtspots behandelten Bereichen des Behandlungsbereichs der Haut unterschieden und entsprechend die noch nicht behandelten Bereiche behandelt.

Alternativ zu dem sequentiellen, individuellen Anfahren einer jeden x/y/z-Koordinate des jeweiligen Lichtspots kann das optische System 4 oder auch die UVA-Lichtquelle 2 und/oder die zweite Lichtquelle 3 gleichzeitig mehrere Lichtstrahlbündel als eine Beam-Matrix erzeugen, die gleichzeitig als die fokussierten Lichtstrahlbündel 51 appliziert werden können. Einzelne Beams bzw. einzelne fokussierte Lichtstrahlbündel 51 können bei einer Lichtspot-Salve bevorzugt auch ausgeblendet bzw. nicht angesteuert werden, falls in dem entsprechenden Fokuspunkt 52 aus physiologischen Gründen kein Lichtspot erzeugt werden soll. Hierbei ist es denkbar, dass das optische System 4 eine multifokale Linse, wie sie durch Altshuler (WO 02/053 050 A1) beschrieben ist, enthält. Bevorzugt umfasst das optische System 4 jedoch mehrere einzelne und in ihren Lagen steuerbare Linsen, die also keine konstanten und vordefinierten Abstände zueinander aufweisen, sondern die durch mehrere jeweils zugeordnete Aktuatoren bzw. Verstellvorrichtungen so angesteuert werden können, dass sie in ihrem jeweiligen Verstellbereich mit dem jeweiligen Fokuspunkt 52 mit einem der bestimmten Lichtspots der Lichtspot-Matrix zur Übereinstimmung kommen. Dadurch läßt sich einerseits die Behandlungszeit wesentlich verkürzen, und es lassen sich, anders als bei Altshuler (WO 02/053 050 A1), bei dem die Lichtspots vordefinierte und unveränderbare Abstände zueinander haben, die Lichtspots gezielt, präzise und individuell dem Gewebe angepasst positionieren. Da bei der gleichzeitigen Applikation der Lichtspot-Salve entsprechend der Beam-Matrix minimale Abstände der Lichtspots bzw. der Beams zueinander vorliegen, die durch die Bauteildurchmesser der LEDs, Dioden-Laser und/oder der Linsen oder durch andere Bauelemente oder Halterungsvorrichtungen bedingt sind, wird bevorzugt nach Applikation einer ersten Lichtspot-Salve das optische System 4 und/oder die erste und zweite Lichtquelle 2 bzw. 3 soweit verschoben bzw. verstellt, dass die jeweils nächstliegenden Lichtspots der Lichtspot-Matrix von der nächsten Lichtspot-Salve abgedeckt werden. Dieser Vorgang wird so oft wiederholt, bis die gesamte Lichtspot-Matrix im Behandlungsbereich abgedeckt ist. Bei einer Beam-Matrix, die aus beispielsweise aus 10x10 Lichtspots besteht, kann die Behandlungszeit bis um den Faktor 100 reduziert werden. Es ist auch denkbar, dass das Lasertherapiesystem anstelle eines verstellbaren, multifokalen Linsensystems verstellbare Lichtquellen aufweist, wobei eine Vielzahl einzelner Lichtquellen im UVA- und/oder im zweiten Wellenlängenbereich nebeneinander gitter- bzw. matrixförmig angeordnet und verstellbar sind.

Bevorzugt werden vor allem gleichzeitig oder semiautomatisch sequentiell mehrere fokussierte Lichtstrahlbündel 51 im ersten Wellenlängenbereich des UVA Lichts als die UVA-Lichtspot-Matrix erzeugt. Alternativ kann die UVA-Lichtquelle 2 auch beispielsweise als eine Dioden-Laser- oder LED-Matrix aufgebaut sein, die gleichzeitig in den diskreten Abständen zueinander die Lichtstrahlbündel erzeugt, die fokussiert oder nicht fokussiert sein können und bevorzugt für jeden Lichtspot einzeln im Verstellbereich positionierbar sind.

Für das zweite Laser-Licht mit der zweiten Lichtwellenlänge gilt das gleiche wie für das UVA-Licht. Auch hierfür kann bevorzugter Maßen eine zweite Laser-Lichtspot-Matrix für das zweite Laser-Licht mit entsprechend vielen parallelen fokussierten Lichtstrahlbündeln 51 bzw. Fokuspunkten 52 erzeugt und angewendet werden, wobei es hier jedoch genauer auf die Positionierung der zweiten Laser-Lichtspots ankommt als bei UVA Licht, so dass bevorzugt mit dem zweiten Laser-Licht eine sequentielle Ansteuerung jedes einzelnen Lichtspots vorgenommen wird.

In Fig. 3a ist in Draufsicht ein schematisiertes Schnittbild einer in der Tiefe der Haut liegenden, papillaren Dermis 22 mit den entsprechenden Papillen 23, die von oben gesehen als rundliche Ränder sichtbar sind, skizziert. Zwischen den Papillen 23 sind Fokuspunkte 52 als sternförmige Lichtspots skizziert, an den Stellen, an denen sie mit der ersten UVA-Wellenlänge erzeugt werden sollen und schließlich erzeugt werden. Rechts daneben ist der vertikale Schnitt des Bereichs der Haut dargestellt, die auch links im Bild in Draufsicht dargestellt ist. Zwischen den im vertikalen Schnittbild skizzierten hügeligen Papillen 23 sind ebenfalls die Fokuspunkte 52 als sternförmige Lichtspots skizziert.

In Fig. 3b ist in Draufsicht das gleiche schematisierte Schnittbild der Haut mit den entsprechenden Papillen 23, wie in Fig. 3a skizziert, dargestellt, jedoch hat sich durch die Applikation der UVA-Lichtspots eine initiale gitterförmige Kollagen- und Elastin-Grundstruktur 25 als eine initiale Kollagen- und Elastin-Matrix zwischen und um die Papillen 23 herum durch das Crosslinking ausgebildet, die punktiert dargestellt ist. Sie gibt für Fibroplasten (Kollagenfaser bildende Zellen) zu einem An- bzw. Ausbau von einem weiteren Kollagen 26 entlang der Kollagen- und Elastin-Grundstruktur 25 Richtungen und eine Netzstruktur vor.

In Fig. 3c ist in Draufsicht das gleiche schematisierte Schnittbild der Haut mit den entsprechenden Papillen 23 und der initialen Kollagen- und Elastin-Grundstruktur 25, wie in Fig. 3b skizziert, dargestellt, jedoch sind zusätzlich die zweiten Fokuspunkte 52 der zweiten Laser Lichtspots als Sterne skizziert. Die zweiten Laser Lichtspots werden möglichst nahe an den Endarteriolen der entsprechenden Papillen 23 positioniert, um so viel wie möglich der durch den Lichtspot abgegebenen Energie auf die Endarteriolen einwirken zu lassen. Die Intensität und die Pulsdauer der Lichtspots ist jeweils der Tiefe der Lichtspots bzw. der Absorption der vorherigen Hautschichten angepasst und daran, dass an den Endarteriolen die gewünschte Energie ankommt. Das Lasertherapiesystem ist bevorzugt so ausgelegt, dass im zweiten Laser-Lichtbereich die zweite Lichtwellenlänge, die zweite Pulsdauer und die zweite Intensität so eingestellt bzw. angesteuert werden kann, dass zwischen einer thermischen, einer photoablativen oder einer plasmainduzierten Wechselwirkung mit dem Gewebe der Haut gewählt werden kann. Ziel ist es, die besagten Parameter so einstellen bzw. steuern zu können, dass die Endarteriolen für eine ausreichend lange Zeit und vorzugsweise für bis zu 20-50 Sekunden permeabel für die korpuskularen Blutbestandteile und dabei bevorzugt für Thrombozyten werden. Dadurch wird die Chemotaxis angeregt, es wandern Fibroblasten ein, und es werden Wachstumsfaktoren z.B. aus den Thrombozyten freigesetzt. Die Wachstumsfaktoren führen zur Induktion des weiteren Kollagens. Im Wesentlichen sind hierbei als bestimmende Wachstumsfaktoren zu nennen: TGF β3, VEGF, EGF, PDGF.

In Fig. 3d ist in Draufsicht das gleiche schematisierte Schnittbild der Haut mit den entsprechenden Papillen 23 und der initialen Kollagen- und Elastin-Grundstruktur 25, wie in Fig. 3c dargestellt, skizziert, jedoch mit weiterem, durch Fibroplasten auf/angebautem Kollagen 26 entlang der initialen Kollagen- und Elastin-Grundstruktur 25 und um die Papillen 23 herum. Dabei nutzen die Fibroblasten die nur schwach ausgebildete Kollagen- und Elastin-Grundstruktur 25 als eine richtungsgebende Struktur und wandern auf dieser entlang, vergleichbar mit einer Spinne im Netz, die ihr Netz ausbessert, oder verstärkt. Bei einer in Fig. 3c dargestellten Behandlung, die beispielsweise einem Laser-Needling entspricht, dauert ein Prozess für einen vollständigen Aufbau der Kollagenstruktur der Haut und damit für eine vollständige Remodellierung eines jugendlichen Hautskelettes zwischen 3 und 6 Monaten.

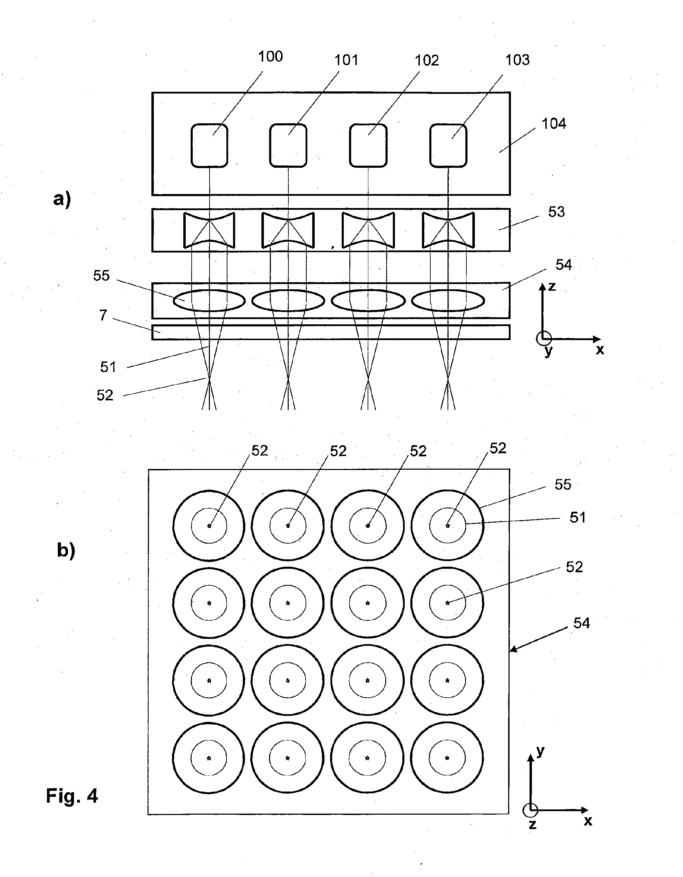

In Fig. 4a ist schematisch in Seitenansicht eine bevorzugte Vorrichtung einer Lichtquelleneinheit 104 mit vier darin integrierten Lichtquellen an den Positionen 100-103 dargestellt, die deren Lichtstrahlenbündel zu einem ersten Linsensystem 53 weiterleitet und daran ankoppelt. Das erste Linsensystem 53 enthält bevorzugt bikonkave Linsen, die die eingehenden Lichtstrahlenbündel aufweiten und entsprechend aufgeweitete jeweilige Lichtstrahlbündel erzeugen. An das erste Linsensystem 53 ist ein zweites Linsensystem 54 angekoppelt, das die aufgeweiteten Lichtstrahlenbündel aufnimmt, jeweils durch eine Linse 55 leitet und mit einem bestimmten Brennpunkt fokussiert, so dass aus den aufgeweiteten Lichtstrahlenbündeln hinter den Linsen 55 jeweils fokussierte Lichtstrahlbündel 51 mit jeweils einem Fokuspunkt 52 erzeugt werden. An den Lichtausgang des zweiten Linsensystems 54 ist die Adapterplatte 7 in einem definierten Abstand angekoppelt, so dass die fokussierten Lichtstrahlbündel 51 hinter der Adapterplatte 7 örtlich definiert austreten. Bei Verschiebung des zweiten Linsensystems in der Tiefe z, werden die Fokuspunkte 52 in gleichem Maße verschoben. So läßt sich also auf einfache Weise die Tiefe z der Fokuspunkte 52 hinter bzw. unter der Adapterplatte 7 verstellen bzw. steuern.

Im Beispiel der Fig. 4a sind an den Positionen 100-103 vier Laserdioden vorstellbar, von denen bevorzugt zwei Laserdioden Licht im UVA-Bereich und zwei Laserdioden Licht im zweiten Wellenlängenbereich abgeben. Beispielsweise sind an den Positionen 100 und 102 UVA-Licht-Dioden und an den Positionen 101 und 103 Laserdioden für das zweite Laser-Licht eingesetzt.

In Fig. 4b ist in Draufsicht vom ersten Linsensystem 53 her gesehen schematisiert das zweite Linsensystem 54 der bevorzugten Vorrichtung aus Fig. 4a dargestellt. Die Anordnung zeigt vier mal vier Linsen in x- und y-Richtung, wobei in jeder Linse 55 als feine Linien die austretenden fokussierten Lichtstrahlbündel 51 und sternförmig die erzeugten Fokuspunkte 52 skizziert sind. Die erzeugten Fokuspunkte 52 hinter dem zweiten Linsensystem 54 stellen in diesem Fall die jeweilige Beam-Matrix des Systems dar.

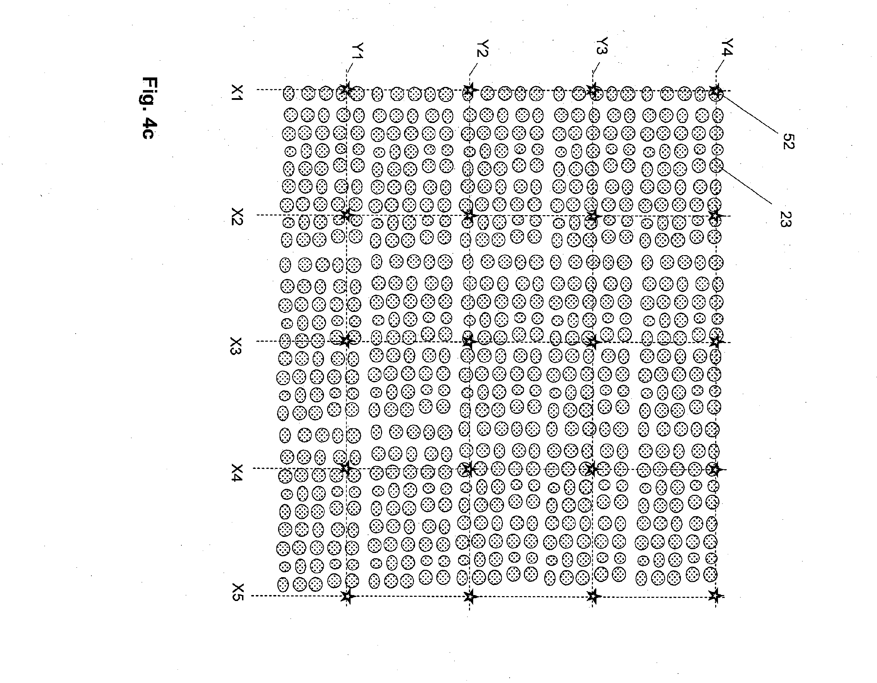

In Fig. 4c ist schematisch ein Schnittbild eines größeren Behandlungsbereichs der Haut mit ihren angedeuteten dermalen Papillen 23 dargestellt. Darauf überlagert sind beispielsweise als Sterne skizziert die Fokuspunkte 52 einer anderen Beam-Matrix dargestellt, wobei jedem Lichtspot der Beam-Matrix eine Koordinate (X1/Y1) - (X5/Y4) zugeordnet ist. Anhand dieses Beispiels soll erläutert werden, dass bei einer fest angeordneten äquidistanten Beam-Matrix gemäß Altshuler (WO 02/053 050 A1) nicht die gesamte Salve an Beams physiologisch richtig liegt, sondern bevorzugt nur ein Teil der Beams, diejenigen nämlich, die zufällig richtig liegen. Im vorliegenden Beispiel würden unter der Annahme, dass ein bestimmter Fang- bzw. Toleranzbereich definiert ist, in dem beispielsweise die zweiten Laser-Lichtspots noch erzeugt werden dürfen, alle Beams bis auf die Beams an den Koordinaten (X2/Y1) - (X5/Y1), (X2/Y2), (X2/Y3), (X5/Y2) - (X5/Y4) als Lichtspots aktiviert werden. Im Falle einer bevorzugten Vorrichtung, bei der die einzelnen Beams um +/- 10% eines Grundabstandes verschoben bzw. verstellt positioniert werden können, kann jeder der Beams physiologisch richtig positioniert werden und einen Lichtspot vor dem gesamten Verschieben der Vorrichtung erzeugen.

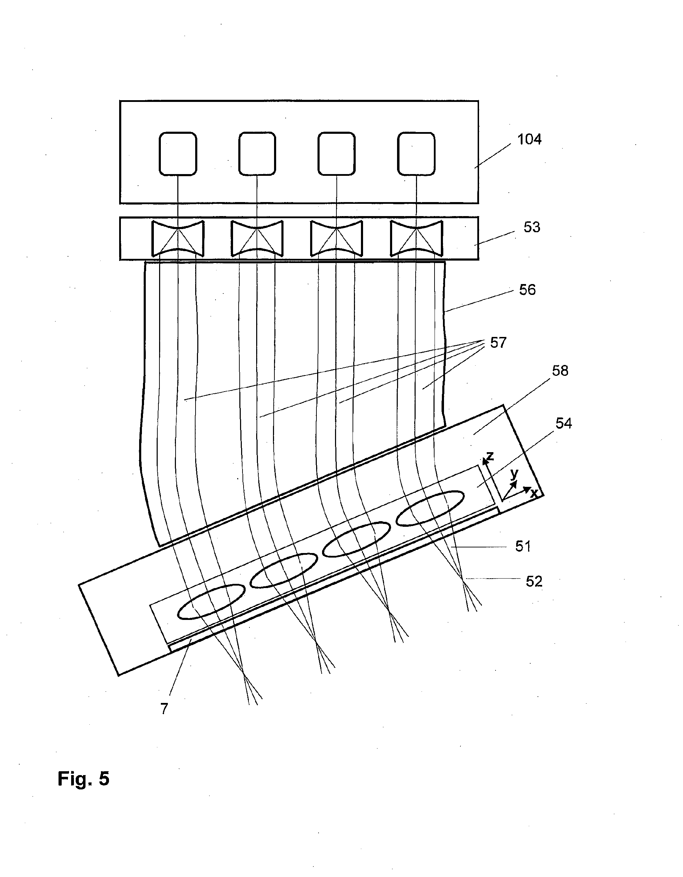

In Fig. 5 ist eine bevorzugte ähnliche Vorrichtung wie in Fig. 4a skizziert dargestellt, die die Lichtquelleneinheit 104 mit den vier darin integrierten Lichtquellen und das daran angekoppelte erste Linsensystem 53 umfasst, aus dem die aufgeweiteten Lichtstrahlbündel austreten und nunmehr in einen Lichtleiter 56 eingekoppelt werden. Der Lichtleiter 56 nimmt die aufgeweiteten Lichtstrahlbündel jeweils durch darin enthaltene Lichtfaserbündel 57 auf, und leitet als flexibler Lichtleiter 56 die aufgeweiteten Lichtstrahlbündel weiter an einen Lichtadapterkopf 58, der die eingehenden, aufgeweitete Lichtstrahlbündel aufnimmt. Der Lichtadapterkopf 58 umfasst das fokussierende zweite Linsensystem 54 und leitet die eingehenden Lichtstrahlen durch das zweite Linsensystem 54 hindurch zu der Adapterplatte 7. Die durch das zweite Linsensystem 54 erzeugten, fokussierten Lichtstrahlbündel 51 treten so hinter der Adapterplatte 7 und aus dem Lichtadapterkopf 58 aus, der auf die Haut aufgelegt wird, so dass die fokussierten Lichtstrahlbündel 51 und die Fokuspunkte 52 räumlich definiert aus dem Lichtadapterkopf 58 austreten und im Behandlungsbereich der Haut positioniert bzw. navigiert werden können. Der wesentliche Vorteil dieses Systems ist die Trennung des Lasertherapiesystems in einen schweren, großen Teil und in einen relativ kleinen, flexiblen und handlichen Teil, der auf die Haut aufgelegt, dort kurzzeitig zur Behandlung fixiert und dann zu einer nächsten Behandlungsstelle weiterverschoben wird. Indem das zweite Linsensystem 54 beispielsweise im Lichtadapterkopf 58 in der Tiefe z verstellt wird, lässt sich der Fokuspunkt 52 hinter der Adapterplatte 7 positionieren bzw. einstellen.

Eine solche Anordnung einer Trennung des ersten schweren, großen Teils des Lasertherapiesystems von dem relativ kleinen, flexiblen und handlichen Teil, der auf die Haut aufgelegt wird, ist ebenso für alle hierin beschriebenen erfindungsgemäßen Vorrichtungen, wie beispielsweise derjenigen aus Fig. 1 oder Fig. 2 denkbar und bevorzugt und nur zwecks Übersichtlichkeit weggelassen. Ein anderer bevorzugter Lichtleiter 56 umfasst anstelle von Lichtfaserbündeln 57 Spiegelsysteme.

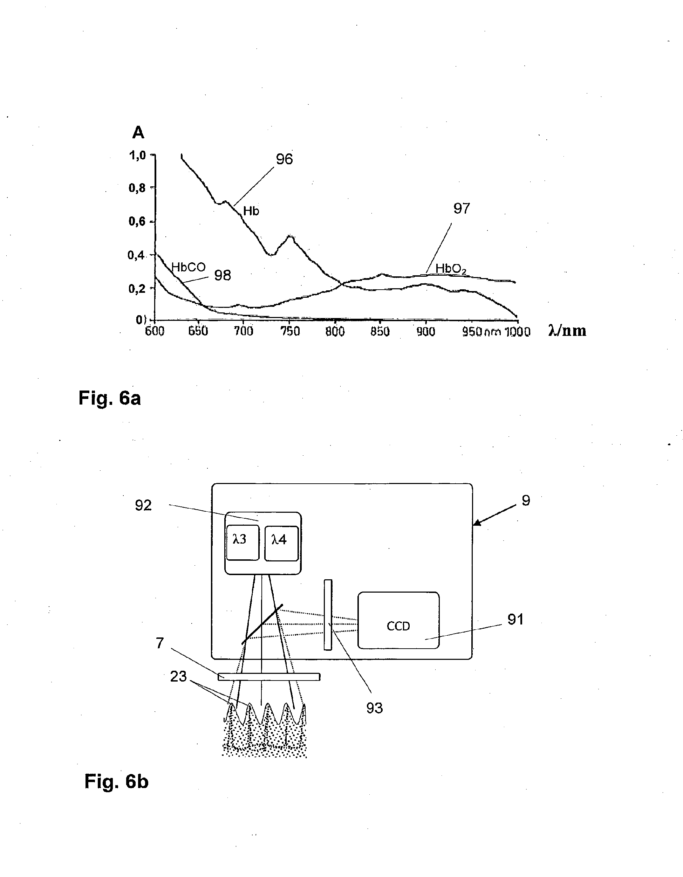

In Fig. 6a sind in einem Diagramm der spezifische Absorptionskoeffizient über der Beleuchtungslichtwellenlänge von oxygeniertem Blut 97 und von desoxygeniertem Blut 96 aufgetragen. Zusätzlich ist der spezifische Absorptionskoeffizient von Kohlenmonoxyd angereichertem Blut 98 aufgetragen. Die Tabelle entstammt dem Buch von J. Eichmeier: Medizinische Elektronik, zweite Auflage. Das Diagramm soll veranschaulichen, dass, wie beispielsweise bei der SpO2-Messung, bei unterschiedlichen Beleuchtungswellenlängen unterschiedliche Stoffe und Gewebeteile besser oder weniger gut spezifisch kontrastiert detektiert werden können. Für die Detektion der Endarteriolen in den Papillen 23 wird bevorzugt ein erstes Bild bei einer ersten Beleuchtungswellenlänge bei 800 nm im isosbestischen Punkt und ein zweites Bild bei einer davon abweichenden zweiten Beleuchtungswellenlänge von z.B. 650-750nm aufgenommen und zusammen verarbeitet. Als Bildverarbeitung wird bevorzugt eine Logarithmierung der Intensitäten des ersten und des zweiten Bildes und eine anschließende Subtraktion voneinander durchgeführt. Weitere Kantenanhebungsalgorithmen lassen dann Endarteriolen besonders kontrastreich erscheinen, wenn es um die Frage geht, wo beispielsweise die Papillen 23 im Behandlungsbereich liegen und wo die zweiten Laser-Lichtspots zu setzen sind. Alternativ kann auch mit Weißlicht oder mit beiden Beleuchtungswellenlängen gleichzeitig beleuchtet werden, wenn ein Bild mit einer Farb-CCD-Kamera aufgenommen wird und danach aus dem Farbbild jeweils ein Bild aus dem Rot-Anteil, ein Bild aus dem Grün-Anteil und ein Bild aus dem Blau-Anteil gewonnen und dann zusammen verarbeitet wird.

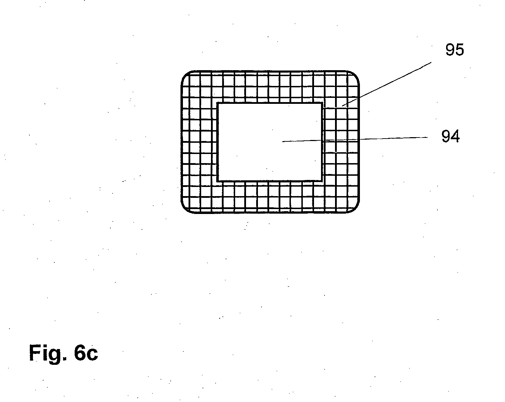

Fig. 6b zeigt eine schematische Darstellung eines Kamerasystems 9, das bevorzugt eine Kamera-Einheit 91, beispielsweise als CCD-Sensor ausgebildet, ein oder mehrere optische Filter 93, einen halbdurchlässigen Spiegel und eine Beleuchtungsquelle 92 umfasst. Der Strahlengang der Beleuchtungsquelle 92 wird über den halbdurchlässigen Spiegel durch die Adapterplatte 7 auf die Haut gerichtet, dort auf der Oberfläche und in der Tiefe reflektiert, wobei die reflektierten Strahlen ebenfalls durch die Adapterplatte 7 hindurchtreten und vom halbdurchlässigen Spiegel reflektiert zur Kamera-Einheit weitergeleitet werden. Die Beleuchtungsquelle 92 umfasst bevorzugt mindestens zwei getrennte Beleuchtungslichtquellen oder eine gemeinsame Beleuchtungslichtquelle, die mindestens die erste Beleuchtungswellenlänge und die zweite Beleuchtungswellenlänge aufweist. Die Beleuchtung der Haut und die Lichtstrahlen des reflektierten, gewonnenen ersten und zweiten Bildes können, wie in Fig. 6b skizziert, mittels eines halbdurchlässigen Spiegels durch ein gemeinsames Objektiv bzw. durch einen gemeinsamen Strahlengang erfolgen. Es ist aber auch denkbar, die Kamera-Einheiten rechts und links der Beleuchtung der Haut zu positionieren, um dabei auch ein Stereo-Bild mit Tiefendarstellung zu gewinnen. Oder die Beleuchtung wird seitlich appliziert, wobei die Bildgewinnung dann zentral erfolgt, wie in Fig. 6c skizziert. In Fig. 6c ist das Detektionsfeld 94 der Kamera-Einheit zentral angeordnet, und die Beleuchtung wird seitlich durch das Beleuchtungsfeld 95 in die Haut eingekoppelt.

Die Bildverarbeitung, die rechentechnisch durch einen oder mehrere Prozessoren oder Elektroniken geschieht, kann ganz oder teilweise im Kamerasystem 9, in einem zusätzlichen Prozessorsystem oder in der Steuereinheit 8 durchgeführt werden.

Darüber hinaus ist es erfindungsgemäß auch denkbar, dass für eine Anwendung, bei der eine Wunde mit zwei getrennten Hautteilen oder eine offene Wunde verschlossen werden soll, das Lasertherapiesystem zusätzlich eine Abgabeeinheit für einen Hautkleber und/oder für ein Wundpflaster umfasst, die darauf abgestimmt sind mit der Lasertherapiesystem zusammen zur Anwendung zu kommen und bei Lichtbestrahlung Reaktionen in der Haut auszulösen.

Der Hautkleber besteht vorzugsweise auch einem Gemisch eines organischen Hautklebers, wie beispielsweise Dihydroxyaceton oder DHA, der mit dem Photosensitizer-haltigen Mittel, wie beispielsweise Riboflavin, zu einem homogenen Gemisch vermengt ist. Der Hautkleber weist eine erste Klebekraft auf, um die zwei getrennten Hautteile zusammenzuhalten. Eine erste Verklebung der Haut und/oder von Gewebeteilen ist alternativ auch mit ionisierten Nanopartikeln gegensätzlicher Polarisierung von einem ersten zu einem zweiten Wundrand denkbar. Der darin enthaltene Photosensitizer bewirkt eine Reaktion in der Haut, bei der durch eine UVA-Lichtspot-Bestrahlung durch das Lasertherapiesystem Kollagen und Elastin vernetzt gebildet wird. Dabei hat das Lasertherapiesystem die vorher beschriebenen Eigenschaften, gezielt positioniert in der Tiefe der Haut die Kollagen- und Elastin-Grundstruktur 25 durch Vernetzung ausbilden zu können, indem die Lichtspots gezielt manuell oder automatisiert navigiert werden können. Darüber hinaus ist es auch vorstellbar, eine intensivere UVA-Licht-Behandlung bei instantan sich bildendem Kollagen und Elastin durchzuführen, um dadurch schneller eine höhere Zugfestigkeit zu erzielen. Eine weitere Behandlung mit den zweiten Laser-Lichtspots der zweiten Wellenlänge kann, wie oben beschrieben, erfolgen.

Das Wundpflaster ist so beschaffen, dass es bevorzugt eine isolierende Schutzfunktion gegen Infektionen aufweist, zudem weist es eine hohe Lichttransparenz auf, um nach Aufbringen des Wundpflasters auf der Wunde für die Lichtstrahlen der ersten und der zweiten Wellenlänge des Lasertherapiesystems transparent zu sein. Ferner weist es eine flexible Eigenschaft auf, um sich einer Hautoberfläche anzupassen, es weist Klebekraft auf, um auf der Haut kleben zu bleiben und es weist eine gewisse Zugfestigkeit auf, um die Hautteile zusammen zu halten. Dabei weist das Wundpflaster bevorzugt Nano-Saugnäpfe an seiner Unterseite auf, um die Klebewirkung herzustellen.

Es ist auch vorstellbar, dass das bevorzugte Wundpflaster auf seiner Unterseite eine Gelschicht aus einem anderen in die Haut einziehenden Photosensitizer-haltigen Mittel aufweist, das in die Haut eindringt und danach mit dem Lasertherapiesystem in der Haut oder auf der offenen Haut zur Reaktion gebracht werden kann, um dadurch eine erste Schutzschicht auf der Haut zu erzeugen.

Wenn erste Blutungen wieder abgeheilt sind, kann das Lasertherapiesystem erneut angewendet werden und unter Anwendung des zweiten Laser-Lichts als Laser-Needling fungieren, um dadurch für eine weitere Ausschüttung der Wachstumsfaktoren zu sorgen und um letztlich den Prozess der Hautregeneration zu komplettieren.

Mit dem Lasertherapiesystem ist es möglich, das "Hautskelett" nicht nur undifferenziert zu induzieren, sondern "physiologisch" und narbenfrei zu regenerieren, wodurch die Haut als Gesamtes in Funktion und Morphologie nicht nur verschweißt, sondern im Sinne einer restitutio ad integrum wiederherstellt wird.

Darüber hinaus ist es erfindungsgemäß auch denkbar, dass das Lasertherapiesystem mit dessen Kamerasystem 9 auch in tieferen Regionen bis 15 mm unter der Haut eingesetzt wird, in denen beispielsweise zunächst mittels UVA-Lichtspots gezielt Kollagen und Elastin zur Verbindung getrennter oder zur Abdeckung von verletzten Gefäßen eingesetzt wird, um diese zu verbinden oder zu stärken, wonach, wie oben beschrieben, bevorzugt die zweiten Laser-Lichtspots appliziert werden, um die Kollagen- und Elastin-Grundstruktur 25, die matrixförmig ausgebildet ist, weiter zu festigen. Auch ist es ebenso denkbar, freigelegte Gefäße oder Gewebe auf diese Art zu verbinden bzw. zu stärken.

Darüber hinaus werden in dem Lasertherapiesystem Kalibrierparameter abgespeichert gehalten, die auch durch einen Kalibrier-Dummy entsprechend aktualisiert werden können.

Ferner weist das Lasertherapiesystem bevorzugt eine Kühleinheit für die Haut auf, die entweder die Adapterplatte (7) selbst oder eine vorgeschaltete hochtransparente Kühlplatte umfasst.

Ebenso ist es denkbar, dass das Lasertherapiesystem für eine Anwendung am Auge adaptiert bzw. ausgebildet ist, bei der die Haut des Auges und dabei insbesondere die Lederhaut bzw. Sclera oder die Bindehaut bzw. Conjunktiva entsprechend behandelt wird. Durch die Anwendung an der Bindehaut kann die Bindehaut wieder gestärkt werden und wird dadurch auch wieder weißer. Durch die Anwendung an der Lederhaut kann die Lederhaut und damit der gesamte Augapfel wieder gestärkt werden, so dass sich das Auge entlang der optischen Achse verkürzt und eine Myopie korrigiert wird.

### Verfahrensbeschreibung und Physiologie

In einer gesunden, jungen Haut findet man im Bereich der Dermis 22 eine natürliche, netzartige Kollagen-Struktur, die der darüber liegenden Epidermis 21 als Hautskelett dient und diese quasi zeltartig ausspannt. Zwischen der Epidermis 21 und der Dermis 22 sind bäumchenartige, verzweigte, orthogonal liegende Elastinfasern und die Grundsubstanz (Proteoglykane, Glykoaminoglykane, usw.) zu finden. Dieser physiologische anatomische Aufbau gibt der jungen Haut die für sie typische, natürliche Elastizität und Festigkeit. Durch Alterungsprozesse löst sich die matrixartige Kollagen- und Elastin-Struktur und das elastische Hautskelett langsam auf und wird durch wenige, verdickte, ungerichtete und unspezifisch verknüpfte, glykosylierte Kollagenfasern ersetzt.

Bei einer Verletzung oder bei einem bis in das dermale Hautniveau reichenden Schnitt entsteht eine diskrete Unterbrechung des Bindegewebes und damit der Kollagen- und Elastin-Struktur.

Eine neuartige Behandlungsmethode zur Hautverjüngung umfasst in einem ersten Behandlungsschritt, die verloren gegangene, natürliche Kollagen- und Elastin-Grundstruktur und Elastinmatrix genau positioniert und definiert zunächst in einem solchen Maße wieder herzustellen, dass sie physiologisch bzw. anatomisch korrekt angeordnet und ausgerichtet ist und gerade genügend ausgebildet ist, um als erstes Kollagen- und Elastin-Grundgerüst bzw. als die richtungsbestimmende initiale Kollagen- und Elastin-Grundstruktur 25 für die Kollagen aufbauenden Fibroplasten zu dienen. Auf diese Weise entsteht die der ursprünglichen, jugendlichen Kollagen- und Elastin-Struktur ähnliche oder identische Kollagen- und Elastin-Grundstruktur 25 mit den damit verbundenen physiologischen Eigenschaften wie Elastizität, Festigkeit und Morphologie.

Der erste Behandlungsschritt umfasst vorzugsweise zunächst die Applikation des die Haut bis in die Dermis 22 penetrierenden, Photosensitizer-haltigen Mittels, das als Creme oder Gel beispielsweise Riboflavin enthält, und das in den Bereich der papillären Dermis 22 in ca. 10 - 30 Minuten einzieht. Nachdem das Mittel genügend tief in den papillaren Bereich eingedrungen ist, werden gezielt zwischen den Papillen bzw. um die Papillen herum die UVA-Lichtspots mit bestimmten Energien appliziert, so dass an den jeweiligen UVA-Lichtspots Kollagen- und Elastinfasern entsprechend der Lysyloxidasereaktion quervernetzt werden (Crosslinking). In anderen Worten katalysiert das Photosensitizer-haltige Mittel das Kollagen- und Elastin-Crosslinking im Sinne einer Aldolkondensation, ähnlich der Lysyloxidasereaktion. Die jeweils nächsten, benachbarten Lichtspots werden jeweils nahe genug an den vorherigen Lichtspots positioniert bzw. erzeugt, um dadurch die verloren gegangene, physiologisch natürliche Kollagen- und Elastin-Grundstruktur 25 gezielt wieder herzustellen, an der dann das weitere Kollagen und Elastin angebaut werden kann. Vorzugsweise wird das UVA-Licht als das fokussiertes Lichtstrahlbündel 51 mit einem Fokuspunkt 52 gepulst erzeugt, wobei der Lichtspot bzw. der Fokuspunkt 52 gezielt an die gewünschte Stelle an der Papille navigiert wird. Dabei ist bei dem gepulsten, fokussierten Lichtstrahlbündel 51 die Eintrittsfläche in die Haut um ein vielfaches größer als die Querschnittsfläche im Fokuspunkt 52, so dass entsprechend die Leuchtdichte im Fokuspunkt 52 um ein Vielfaches größer ist, als beim Lichteintritt in die Haut. Die Lichtintensität und die Pulsdauer des UVA-Lichts im Fokuspunkt 52 werden so eingestellt, dass gerade sicher die Kollagen- und Elastin-Bildung im Lichtspot entsteht. Bei einer Lichtpuls-Erzeugung können auch gleichzeitig viele nebeneinander liegende Lichtpulse erzeugt werden, sofern diese physiologisch sinnvoll liegen, um damit eine Behandlungszeit zu reduzieren. Da UVA-Licht leider auch eine zytotoxische Wirkung aufweist, wird damit so sparsam wie möglich umgegangen, so dass gerade nur eine so dünne Kollagen- und Elastin-Grundstruktur 25 erzeugt wird, die gerade ausreicht, als richtungsgebendes Grundgerüst für später induziertes Kollagen und Elastin zu dienen. Die Energie des UVA- Lichtspots wird so gering gehalten, dass sie vollständig vom Photosensitizer absorbiert werden und damit punktuell genau zur Wirkung kommen, bei minimalen bis keinen Kollateralschäden. Vorzugsweise wird die Applikation des UVA-Lichts bzw. das Positionieren der Lichtspots im Behandlungsbereich um die Papillen herum durch die bildgebende Einheit unterstützt, wie beispielsweise durch die Optische KohärenzTomographie (OCT), mit der die Haut und deren Behandlungsbereich bis in die Tiefe hinein dreidimensional dargestellt wird, um beispielsweise die Papillen 23, die Gefäße 24, Nerven, Haarwurzeln, Talgdrüsen und dergleichen dargestellt zu bekommen und die Lichtspots somit gezielter setzen zu können. Mit einer einfacheren bevorzugten Behandlungsvorrichtung bzw. einem Lasertherapiesystem ohne integrierte, bildgebende Einheit wird die Lichtspotmatrix nach Einstellung des entsprechenden Hauttyps mit einem vordefinierten Raster in der Tiefe der Haut erzeugt. Eine weitere bevorzugte Behandlungsvorrichtung misst durch ein einfacheres, reflexionsphotometrisches oder diaskopisches Verfahren nur die Tiefe und Lage der papillaren Schicht, ohne die Haut im Detail darzustellen, um die Erzeugung der Lichtspotmatrix in der richtigen, bestimmten Tiefe und an den richtigen Stellen auszuführen. Alternativ zu OCT kann auch das fluoreszenzmikroskopische oder fluoreszenztomographische, bildgebende Verfahren eingesetzt werden, um sich ein Bild von in der Haut liegenden Strukturen, wie beispielsweise den Papillen, Arteriolen, Kollagenfasern oder Teilen davon, zur Ansteuerung der entsprechenden Lichtspots zu verschaffen.

Durch den zweiten Behandlungsschritt wird Laser-induziert die Bildung des weiter in die Kollagen- und Elastin-Grundstruktur 25 einwachsenden und quervernetzenden Kollagens und Elastins angeregt. Dazu wird entweder das Laser-Needling durchgeführt oder eine weniger lichtintensive Laser-Punktierung so vorgenommen, dass die Endarteriolen in der papillären Dermis, also in der Zielregion des Behandlungsbereichs der Haut für korpuskuläre Blutbestandteile wie beispielsweise für Thrombozyten permeabel gemacht werden. Hierdurch kommt es zu einer Freisetzung von Wachstumsfaktoren und Cytokinen, wodurch Fibroblasten aktiviert werden. Infolge der Aktivierung der Fibroblasten wird ein Aufbau weiteren Kollagens und Elastins entlang der zuvor erzeugten Kollagen- und Elastin-Grundstruktur 25 erzeugt. Hierfür wird bevorzugt die gepulste zweite Lichtquelle 3 eingesetzt, mit der bevorzugt größere Energien als bei der UVA-Lichtbehandlung im Lichtspot abgegeben werden. Auch hierbei werden durch Linsen fokussierte Lichtstrahlbündel 51 eingesetzt, die mit der ersten Querschnittsfläche und mit der ersten Leuchtdichte in die Haut eintreten und die im Fokuspunkt in der Tiefe der Haut eine um ein Vielfaches kleine Querschnittsfläche und eine um ein Vielfaches größere Leuchtdichte aufweisen als beim Eintritt in die Haut. Eine Brennweite, die Lichtintensität und die Pulsdauer sind so bemessen, dass der Lichtspot in der richtigen Tiefe der Haut liegt, dass dort die abgegebene Energie für die Therapieanwendung groß genug ist und dass dabei die Hautoberfläche nicht perforiert bzw. möglichst wenig beschädigt wird. Auch bei diesem Verfahren ist die Unterstützung der OCT-Einheit oder einer anderen bildgebenden Einheit von Vorteil, um die Lichtspots in der richtigen Tiefe der Haut an den jeweiligen Endarteriolen positionieren bzw. erzeugen zu können. Andersartige bildgebende Verfahren sind dabei ebenso vorstellbar.

Eine weitere neuartige Behandlungsmethode stellt eine Verbesserung eines Wundverschlusses und einer Wundheilung dar, indem in einem ersten Behandlungsschritt zunächst die Hautteile richtig aneinandergefügt, die Wunde verschlossen und dann die Bildung der initialen Kollagen- und Elastin-Grundstruktur 25 durch gezielte UVA-Lichtspots und damit verbundenem Kollagen-/Elastin Crosslinking vorgenommen wird. Vorzugsweise kann zur Verstärkung des Wundverschlusses auch ein hochtransparentes Pflaster auf die Wunde aufgebracht werden, das für die UVA- und für die zweiten Laser-Lichtspots genügend transparent ist. Dabei ist es vorstellbar, dass das Wundpflaster auch zusätzliche medikamentöse Bestandteile enthält, die einen Heilungsprozess fördern. Anstelle des hochtransparenten Pflasters oder auch zusätzlich ist die Verwendung eines organischen Hautklebers vorstellbar, wie z.B. Dihydroxyaceton oder DHA, der mit Riboflavin zu einem homogenen Gemisch vermengt ist, um die Hautteile aneinander zu kleben. Ähnlich wie oben erwähnt, wird die Hautstruktur mittels OCT dargestellt und es werden gezielt die UVA-Lichtspots appliziert, um die Vernetzung des einen mit dem anderen Hautteil als die Kollagen- und Elastin-Grundstruktur 25 vorzunehmen. Durch die erzeugten Brückenstellen zwischen dem einen und dem anderen Hautteil werden eine Orientierung und ein Einwachsen von weiterem Kollagen und Elastin vorgegeben. Dabei können durch ein späteres, zusätzliches Laser-Needling mit der zweiten Wellenlänge, nachdem die erste Blutung wieder zurückgegangen ist, weitere Mikroblutungen am Wundgrund oder am Wundrand und damit die Ausschüttung weiterer Wachstumsfaktoren erzeugt werden.

Denkbar sind auch die Erzeugung von beispielsweise Organskeletten oder Grundstrukturen anderer Gewebe im Sinne von Tissue Engineering aus beispielsweise rekombinant erzeugtem Kollagen. Hierzu wird beispielsweise mittels einer hochauflösenden optischen Kohärenztomographie (OCT) oder eines anderen bildgebenden Verfahrens ein Gewebe gescannt und dann entsprechend gewebespezifisch mit Lichtspots bearbeitet, um die entsprechenden Kollagen - Crosslinks zu erzeugen. Es ist auch denkbar, diskontinuierte Gewebestrukturen wie beispielsweise durchtrennte Gefäße, periphere oder zentrale Nerven auf diese Weise wieder zu verbinden.

Zusammen mit dem Lasertherapiesystem gemäß einem oder mehreren der nachstehenden Ansprüche wird auch das neuartige Verfahren zur Behandlung der Haut vorgestellt, wobei in dem ersten Schritt zunächst das Photosensitizer-haltige Mittel auf die Haut aufgetragen wird. Nach dessen Hautpenetration vor allem des volumenartigen Behandlungsbereichs werden gezielt an den Stellen der papillären Dermis zwischen und um die Papillen 23 herum die UVA-Lichtspots der ersten Lichtwellenlänge punktgenau nebeneinander und entlang netzartiger Bahnen erzeugt, wodurch in dem papillaren Hautbereich instantan die Kollagen- und Elastin-Grundstruktur gebildet wird. Die UVA-Lichtspots werden so erzeugt und in die Haut appliziert, dass die Leuchtdichte beim Eintritt mit der ersten Querschnittsfläche in die Haut um ein Vielfaches kleiner als im Lichtspot ist, so dass die Oberhaut dadurch weniger im Mitleidenschaft gezogen und vor allem nicht perforiert wird.

In dem zweiten Schritt werden gezielt in den Papillen des Behandlungsbereichs die zweiten Laser-Lichtspots mit der zweiten Lichtwellenlänge erzeugt. Dabei werden die Brennweite des fokussierten Lichtstrahlbündels 51, die Pulspausezeit und die Energie so eingestellt, dass durch den Lichtspot in der Papille 23 die Endarteriolen für die korpuskularen Blutbestandteile permeabel werden. Die zweiten Lichtspots werden dabei so erzeugt und in die Haut appliziert, dass die Leuchtdichte beim Eintritt mit der ersten Querschnittsfläche in die Haut um ein Vielfaches kleiner als im Lichtspot ist, so dass die Oberhaut bzw. die Epidermis 21 dadurch weniger in Mitleidenschaft gezogen und vor allem nicht perforiert wird.

Weitere mögliche Ausbildungsformen sind in den folgenden Ansprüchen beschrieben.

Die In den Ansprüchen genannten Bezugszeichen dienen der besseren Verständlichkeit, beschränken aber die Ansprüche nicht auf die in den Figuren dargestellten Formen.

### Bezugszeichenliste

- 1: Abgabeeinheit zur Abgabe eines Mittels
- 10: Monitor
- 10a: erste Darstellung
- 10b: zweite Darstellung
- 100-103: Positionen
- 104: Lichtquelleneinheit
- 2: UVA-Lichtquelle
- 21: Epidermis
- 22: Dermis
- 23: Papille
- 24: Blutgefäße
- 25: Kollagen- und Elastin-Grundstruktur
- 26: weiteres Kollagen
- 3: zweite Lichtquelle
- 4: optisches System
- 5: Lichtstrahlumlenk- und Fokussieroptik
- 51: fokussiertes Lichtstrahlbündel
- 52: Fokuspunkt
- 53: erstes Linsensystem
- 54: zweites Linsensystem (zur Lichtstrahlfokussierung)
- 55: Linse
- 56: Lichtleiter
- 57: Lichtfaserbündel
- 58: Lichtadapterkopf
- 6: elektromechanische Verstellvorrichtung
- 7: Adapterplatte
- 8: Steuereinheit
- 81: Navigationssteuergerät
- 9: Kamerasystem
- 91: Kamera-Einheit
- 92: Beleuchtungsquelle
- 93: optisches Filter
- 94: Detektionsfeld
- 95: Beleuchtungsfeld
- 96-98: Absorptionskoeffizient-Kennlinien

## Patentansprüche

1. Lasertherapiesystem zur Hautbehandlung in einem volumenartigen Behandlungsbereich in einer Tiefe der Haut, umfassend:
a) eine Abgabeeinheit (1) zur Abgabe eines Photosensitizer-haltigen Mittels zur Applikation auf der Haut;
b) eine erste Lichtquelle (2), die mindestens ein erstes Lichtstrahlbündel mit einer ersten Wellenlänge in einem UVA Bereich erzeugt, deren erste Lichtintensität und erste Lichtpulsdauer steuerbar ist, wobei die erste Wellenlänge, die erste Lichtintensität und die erste Lichtpulsdauer so ausgelegt und steuerbar sind, dass sich in einem Fokuspunkt (52) des fokussierten Lichtstrahlbündels (51) in der Haut in Anwesenheit des Photosensitizer-haltigen Mittels Kollagen- und Elastin-Crosslinks bilden;
c) eine zweite Lichtquelle (3) als Laser-Lichtquelle, die mindestens ein zweites Lichtstrahlbündel mit einer zweiten Wellenlänge erzeugt, deren zweite Lichtintensität und zweite Lichtpulsdauer steuerbar ist, wobei die zweite Wellenlänge, die zweite Lichtintensität und die zweite Lichtpulsdauer so ausgelegt und steuerbar sind, dass in dem Fokuspunkt (52) des fokussierten Lichtstrahlbündels (51) die Endarteriolen zentral im Bereich einer jeweiligen Papille (23) des Stratum Papillare des Behandlungsbereichs für korpuskuläre Blutbestandteile permeabel werden ohne dabei eine darüber liegende Epidermis (21) zu verletzen oder zu perforieren;
d) ein optisches System (4),
das mit der ersten (2) und mit der zweiten Lichtquelle (3) fest verbunden ist und mit dem das mindestens eine erste und zweite Lichtstrahlbündel aufgenommen werden;
umfassend eine Lichtstrahlumlenk- und Fokussier-Optik (5), die eine elektromechanische Verstellvorrichtung (6) und eine hochtransparente Adapterplatte (7) als Lichtaustrittsöffnung aufweist, wobei die Lichtstrahlumlenk- und Fokussier-Optik (5) so und durch die elektromechanische Verstellvorrichtung (6) elektrisch steuerbar verstellbar ausgebildet ist, das aufgenommene jeweilige erste und zweite Lichtstrahlbündel derart weiterzuleiten, umzuleiten und mit einer bestimmten Brennweite fokussiert zu positionieren, dass das jeweilige resultierende fokussierte Lichtstrahlbündel (51) hinter der Adapterplatte (7) mit einer ersten Lichtquerschnittsfläche austritt, und dass dessen jeweiliger Fokuspunkt (52) hinter der Adapterplatte (7) mit x/y/z-Koordinaten punktgenau bestimmt ist und in Bezug auf die Adapterplatte (7) in dem volumenartigen Behandlungsbereich gesteuert punktgenau positionierbar ist, und
e) eine Steuereinheit (8)
zur Ansteuerung der elektromechanischen Verstellvorrichtung (6) des optischen Systems (4) für die Navigation des Fokuspunktes , und
zur Ansteuerung der ersten (2) und der zweiten Lichtquelle (3), so dass die jeweilige Lichtquelle (2, 3) die erste oder die zweite Lichtintensität bei der ersten oder zweiten Lichtpulsdauer erzeugt, um dadurch in den jeweiligen Fokuspunkten (52) gewünschte Lichtspots für eine jeweilige Reaktionen wie das Kollagen- und Elastin-Crosslinking bzw. eine Permeabilität der Endarteriolen für die korpuskularen Blutbestandteile zu erzeugen.

2. Lasertherapiesystem gemäß Anspruch 1, wobei das Lasertherapiesystem zusätzlich eine bildgebende Einheit mit einem Kamerasystem (9) und einen Monitor (10) umfasst, wobei eine Kamera-Einheit (91) des Kamerasystems (9) an das optische System (4) angeschlossen ist und ein Detektionsfeld (94) aufweist, das über einen Strahlengang des optischen Systems (4) den Behandlungsbereich hinter der Adapterplatte (7) abdeckt,
wobei ein am Monitor (10) dargestelltes Bild des Behandlungsbereichs mit x/y/z-Koordinaten mit den x/y/z-Koordinaten des optischen Systems (4), der Steuereinheit (8) und letztlich der Fokuspunkte (52) so übereinstimmend abgleichbar ist, dass der Lichtspot im Behandlungsbereich genau an einer angezeigten Stelle im Bild erzeugbar ist,
und/oder
wobei die Steuereinheit (8) ausgelegt ist, bei der Berechnung der x/y/z-Koordinaten der Fokuspunkte (52) im Behandlungsbereich hinter der Adapterplatte (7) die Lichtbrechung der Haut und eine etwaige zusätzliche Folie oder ein Wundpflaster zwischen der Adapterplatte (7) und der Haut mit zu berücksichtigen.

3. Lasertherapiesystem gemäß Anspruch 2, wobei das Kamerasystem (9) eine optische Kohärenztomographie (OCT), eine Fluoreszenzmikroskopie oder eine Fluoreszenztomographie umfasst und eine dreidimensionale Darstellung des Behandlungsbereichs der Haut erlaubt und Kollagen-Fasern (25, 26), Elastin, Haarwurzeln, Papillen (23), Arteriolen, Endarteriolen, behandeltes Hautgewebe, noch nicht behandeltes Hautgewebe oder Kombinationen daraus sichtbar macht;
oder
wobei das Kamerasystem (9) auf einer Lichtreflexionsmessung oder auf einer Extinktionsmessung mittels der Kamera-Einheit (91) beruht, wobei für eine Bildaufnahme eine Beleuchtung mit einer ersten Beleuchtungswellenlänge wählbar ist, die in arteriellen Bereichen der Haut besonders stark absorbiert oder reflektiert wird, so dass ein arteriell durchbluteter Bereich und damit die Arteriolen und Endarteriolen besonders kontrastiert detektiert und im Bild dargestellt werden;
oder
wobei das Kamerasystem (9) für die Lichtreflexionsmessung mittels Kamera-Einheit (91) eine Beleuchtungsquelle (92) mit mindestens zwei unterschiedlichen Beleuchtungswellenlängen vorsieht, wobei in einem ersten Schritt mindestens zwei Kamera-Bilder bei den unterschiedlichen Beleuchtungswellenlängen erzeugbar und abspeicherbar sind, wobei die jeweilige Beleuchtungswellenlänge auf die hautsächlich zu detektierenden und zu kontrastierenden Hautbestandteile abstimmbar ist, und wobei eine der Beleuchtungswellenlängen gleich oder ungleich der ersten oder der zweiten Lichtwellenlänge ist, und wobei in einem zweiten Schritt durch eine Bildverarbeitung aus den mindestens zwei abgespeicherten Kamera-Bildern das Bild des Behandlungsbereichs erzeugbar und darstellbar ist, wobei die Bildverarbeitung die zwei Kamera-Bilder addiert, von einander subtrahiert, logarithmiert und dann von einander subtrahiert, zusätzlich eine Kantenanhebung vornimmt oder die Kamera-Bilder RGB-farbselektiv auswertet.

4. Lasertherapiesystem gemäß Anspruche 2 oder 3, wobei das Bild den Behandlungsbereich hinter der Adapterplatte (7) anzeigt, und wobei mit einer Prozessoreinheit auf Basis einer Mustererkennung der papillare Bereich mit den Papillen (23) erkennbar und die Positionen bestimmbar sind, die im ersten und/oder im zweiten Wellenlängenbereich mit Lichtspots zu behandeln sind und zusammen die Lichtspot-Matrix ergeben;
und/oder
wobei einer der Prozessoren des Lasertherapiesystems durch eine zweite kontinuierliche Mustererkennung in dem Bild des Behandlungsbereichs hinter der Adapterplatte (7) ein mögliches Verschieben der Adapterplatte (7) auf der Haut erkennt und darüber hinaus erkennt, welcher Bereich des Behandlungsbereichs schon behandelt wurde und welcher unbehandelte Bereich des Behandlungsbereichs noch zu behandeln ist, wobei der behandelte und der unbehandelte Bereich des Behandlungsbereichs am Monitor (10) unterschiedlich eingefärbt darstellbar ist, und wobei eine entsprechende automatisierte Lichtspot-Erzeugung vornehmbar ist.

5. Lasertherapiesystem gemäß einem oder mehreren der vorstehenden Ansprüche, wobei das Photosensitizer-haltige Mittel eine Creme oder ein Gel mit hautpenetrierender und Kollagen- und Elastin-bildungsfördernder Wirkung ist und das Stoffe wie Riboflavin (Vitamin B2) und Coenzyme für eine Synthese von Kollagen und Elastin wie Vitamin A, Vitamin E und/oder Vitamin C enthält,
und/oder wobei die Abgabeeinheit (1) zur Abgabe des Photosensitizer-haltigen Mittels eine Tube, eine Dose, eine Dosiereinheit, ein Gel-Pflaster oder ein Applikator mit Einwegspritze ist,
und/oder wobei das Photosensitizer-haltige Mittel fluoreszierend ist, um dadurch die Eindringtiefe in der Haut zu bestimmen,
und/oder wobei das Lasertherapiesystem zusätzlich einen Alarm-Timer aufweist, der darauf hinweist, dass das Mittel genügend in die Haut eingezogen ist.

6. Lasertherapiesystem gemäß einem oder mehreren der vorstehenden Ansprüche, wobei die erste Lichtquelle (2) mindestens einen UVA Laser oder eine UVA Lampe mit der ersten Wellenlänge im Bereich von 340 - 450 nm umfasst.

7. Lasertherapiesystem gemäß einem oder mehreren der vorstehenden Ansprüche, wobei die zweite Lichtquelle (3) mindestens einen IR-Laser mit der zweiten Wellenlänge in einem Bereich von 800 - 2000 nm umfasst.

8. Lasertherapiesystem gemäß einem oder mehreren der vorstehenden Ansprüche 1-6, wobei die zweite Lichtquelle (3) einen Laser oder eine Vielzahl an Laserdioden mit der zweiten Wellenlänge in einem Bereich von 450-550nm oder 550-650nm oder von 650-800nm umfasst und darin bevorzugt so gewählt ist, dass sie von Hämoglobin möglichst gut und von einem restlichen Gewebe der Haut möglichst wenig absorbiert wird.

9. Lasertherapiesystem gemäß einem oder mehreren der vorstehenden Ansprüche, wobei die erste (2) und/oder die zweite Lichtquelle (3) mehrere einzelne Lichtquellen umfasst, die in bestimmten Abständen angeordnet sind, um zugleich mehrere erste- und/oder zweite Lichtstrahlbündel zu erzeugen, so dass diese zeitgleich an unterschiedlichen Stellen des Behandlungsbereichs applizierbar sind.

10. Lasertherapiesystem gemäß einem oder mehreren der vorstehenden Ansprüche, wobei das optische System (4) mit dessen Lichtstrahlumlenk- und Fokussier-Optik (5) mindestens eine Linse (55), eine konkave Linse, eine Zylinderlinse, eine Streulinse, eine Fresnel-Linse, eine Flüssigkeitslinse, ein erstes Linsensystem (53), ein zweites Linsensystem (54), einen Lichtleiter (56) ein Lichtleiterfaserbündel (57), einen Lichtadapterkopf (58) oder eine Kombinationen daraus umfasst,
und/oder
wobei die Querschnittsfläche im Fokuspunkt (52) um ein Vielfaches kleiner als die erste Querschnittsfläche beim Lichteintritt in die Haut ist, wobei das Vielfache 3-1000 beträgt;
und/oder
wobei das optische System (4) eine Vielzahl von darin angeordneten Lichtstrahlumlenk- und Fokussier-Optiken (5) umfasst, die ausgebildet sind, eine entsprechende Vielzahl an fokussierten Lichtstrahlbündeln (51) und Fokuspunkten (52) mit entsprechenden Lichtspots hinter der Adapterplatte (7) und in dem Behandlungsbereich gleichzeitig zu erzeugen.

11. Lasertherapiesystem gemäß einem oder mehreren der vorstehenden Ansprüche, wobei die Fokuspunkte (52) der fokussierten Lichtstrahlbündel (51) der ersten Lichtwellenlänge mit den Fokuspunkten (52) der fokussierten Lichtstrahlbündel (51) der zweiten Lichtwellenlänge übereinstimmen, abgesehen von Abweichungen durch unterschiedliche Brechungsindizes bei den unterschiedlichen Wellenlängen, oder wobei die Fokuspunkte (52) der fokussierten Lichtstrahlbündel (51) der ersten Lichtwellenlänge von den Fokuspunkten (52) der fokussierten Lichtstrahlbündel (51) der zweiten Lichtwellenlänge um einen definierten Versatz versetzt sind.

12. Lasertherapiesystem gemäß einem oder mehreren der vorstehenden Ansprüche, wobei die elektromechanische Verstellvorrichtung (5) Aktuatoren umfasst, die zwecks einer Lichtstrahlumlenkung und/oder einer Fokuspunktverstellung mit Umlenkspiegeln, halbdurchlässigen Spiegeln, mit Linsensystemen (53, 54), Blenden, Verschiebeelementen, Lichtleitern (56), optischen Filtern (93) oder Kombinationen daraus verbunden sind, um die jeweiligen Fokuspunkte (52) im Behandlungsbereich gezielt in x/y/z-Richtung positionieren zu können.

13. Lasertherapiesystem gemäß einem oder mehreren der vorstehenden Ansprüche, wobei die Steuereinheit (8) eingerichtet ist, die Lichtspots mit der ersten und/oder mit der zweiten Wellenlänge hinter der Adapterplatte (7) im Behandlungsbereich der Haut automatisiert entlang einer vordefinierten oder einer vorher bestimmten Lichtspot-Matrix zu erzeugen, wobei die Lichtspots entweder durch mindestens zwei diskrete gleichzeitige Lichtspots erzeugt werden oder durch eine sequentielle Abfolge einzelner Lichtspots erzeugt werden;
und/oder
wobei das Lasertherapiesystem Kalibrierparameter abgespeichert hält und/oder, wobei vor einer Behandlung das Lasertherapiesystem durch einen Kalibrier-Dummy kalibrierbar ist;
und/oder
wobei die Adapterplatte (7) selbst oder eine vorgeschaltete hochtransparente Kühlplatte eine Kühlwirkung besitzt und dafür geschaffen ist, direkt auf die Haut über dem darunterliegenden Behandlungsbereichs aufgebracht zu werden.

14. Lasertherapiesystem gemäß einem oder mehreren der vorstehenden Ansprüche, wobei das Lasertherapiesystem zusätzlich eine Abgabeeinheit für einen Wundkleber und/oder für ein Wundpflaster umfasst.

15. Lasertherapiesystem gemäß einem oder mehreren der vorstehenden Ansprüche, wobei
a) die Abgabeeinheit (1) zur Abgabe des Photosensitizer-haltigen Mittels angepasst ist zur Behandlung der Bindehaut oder der Lederhaut des Auges;
b) die erste Lichtquelle (2), mit der ersten Lichtintensität und der ersten Lichtpulsdauer angepasst ist zur Behandlung der Bindehaut oder der Lederhaut des Auges;
c) die zweite Lichtquelle (3) mit der zweiten Lichtintensität und der zweiten Lichtpulsdauer angepasst ist zur Behandlung der Bindehaut oder der Lederhaut des Auges;
d) das optische System (4) mit der Lichtstrahlumlenk- und Fokussier-Optik (5) der elektromechanischen Verstellvorrichtung (6), und der Adapterplatte (7) angepasst ist zur Behandlung der Bindehaut oder der Lederhaut des Auges; und
e) die Steuereinheit (8) angepasst ist zur Behandlung der Bindehaut oder der Lederhaut des Auges.

## Claims

1. Laser therapy system for skin treatment in a volume-like treatment area in a depth of the skin, comprising
a) a dispense unit (1) for dispensing a photosensitizer containing agent for application on the skin;
b) a first light source (2) which creates at least a first light-beam bundle with a first wavelength in an UVA range, whose first light intensity and first light pulse duration are controllable, wherein the first wavelength, the first light intensity and the first light pulse duration are adapted and controllable such that in one focal point (52) of the focused light-beam bundle (51) in the skin, collagen and elastin crosslinks are generated in the presence of the photosensitizer containing agent;
c) a second light source (3) as a laser light source, which creates at least one second light-beam bundle having a second wavelength, whose second light intensity and second light-pulse duration are controllable, wherein the second wavelength, the second light intensity and the second light pulse duration are adapted and controllable such that in the focal point (52) of the focused light-beam bundle (51), the endarterioles become permeable for corpuscular blood components centrally in the area of a respective papilla (23) of the stratum papillare of the treatment area without injuring or perforating an epidermis (21) located above it;
d) an optical system (4)
which is integrally connected to the first (2) and to the second light source (3) and with which the at least one first and second light-beam bundles are captured,
comprising a light-beam deflection and focusing optical system (5) having an electromechanical adjusting device (6) and a highly transparent adapter plate (7) as light emission opening, wherein the light-beam deflection and focusing optical system (5) is adapted such and adjustable electrically controllably by the electromechanical adjusting device (6), to forward the respective first and second light-beam bundles received, deflect and position them in such a way focused with a certain focal length that the respective resulting focused light-beam bundle (51) exits behind the adapter plate (7) with a first light cross-section and such that its respective focal point (52) behind the adapter plate (7) is defined precisely with x/y/z coordinates and can be precisely positioned with reference to the adapter plate (7) in the volume-like treatment area; and
(e) a control unit (8)
for controlling the electromechanical adjusting device (6) of the optical system (4) for navigation of the focal point; and
for controlling the first (2) and the second light source (3) so that the respective light source (2, 3) creates the first or the second light intensity with the first or the second light pulse duration so as to create desired light spots in the respective focal points (52) for a corresponding reaction such as the collagen and elastin cross-linking, or a permeability of the endarterioles for the corpuscular blood components, respectively.

2. Laser therapy system according to Claim 1, the laser therapy system additionally comprising an imaging unit with a camera system (9) and a monitor (10), a camera unit (91) of the camera system (9) being connected to the optical system (4) and having a detection field (94) covering the treatment area behind the adapter plate (7) via an optical path of the optical system (4),
wherein an image of the treatment area shown on the monitor (10) with x/y/z coordinates can be adapted to the x/y/z coordinates of the optical system (4), the control unit (8) and finally the focal points (52) with such agreement that the light spot in the treatment area can be created in the image precisely at an indicated position,
and/or
wherein the control unit (8) is adapted, during calculation of the x/y/z coordinates of the focal points (52) in the treatment area behind the adapter plate (7), to take into account the light refraction of the skin and any additional sheet or band-aid between the adapter plate (7) and the skin.

3. Laser therapy system according to Claim 2, the camera system (9) comprising an optical coherence tomography (OCT), a fluorescence microscopy or a fluorescence tomography and allowing a three-dimensional representation of the treatment area of the skin and visualizing collagen fibers (25, 26), elastin, hair roots, papillae (23), arterioles, endarterioles, treated skin tissue, not yet treated skin tissue or combinations thereof,
or
the camera system (9) being based on a light reflection measurement or on an extinction measurement by means of the camera unit (91), wherein for an image recording, an illumination with a first illumination wavelength is selectable which is particularly strongly absorbed or reflected in arterial areas of the skin so that an area with arterial blood perfusion, and thus the arterioles and endarterioles, are detected and represented in the image with particularly strong contrast,
or
the camera system (9) envisaging, for light reflection measurement by means of the camera unit (91), an illumination source (92) with at least two different illumination wavelengths, where in a first step, at least two camera images can be created and stored at the different illumination wavelengths, wherein the respective illumination wavelength can be adapted to the skin components to be mainly detected and contrasted, and wherein one of the illumination wavelengths is equal or non-equal to the first or to the second light wavelength, and wherein in a second step, the image of the treatment area can be created and displayed by image processing of the at least two stored camera images, wherein image processing adds the two camera images up, subtracts them from one another, takes the logarithm of them and then subtracts them from one another, additionally performs edge enhancement or evaluates the camera images with RGB color selection.

4. Laser therapy system according to Claim 2 or 3, wherein the image indicates the treatment area behind the adapter plate (7) and wherein by a processor unit, on the basis of a pattern recognition, the papillary area with the papillae (23) can be recognized and the positions determined which are to be treated with light spots in the first and/or in the second wavelength range and which together form the light spot matrix,
and/or
wherein one of the processors of the laser therapy system recognizes a possible shift of the adapter plate (7) on the skin by a second continuous pattern recognition in the image of the treatment area behind the adapter plate (7) and additionally recognizes which part of the treatment area has already been treated and which untreated part of the treatment area still has to be treated, wherein the treated and the untreated part of the treatment area can be represented in different colors on the monitor (10), and wherein a corresponding automated light-spot creation can be performed.

5. Laser therapy system according to one or more of the preceding claims, the photosensitizer containing agent being a cream or a gel with skin-penetrating and the formation of collagen and elastin boosting effect and containing substances such as riboflavin (vitamin B2) and coenzymes for a synthesis of collagen and elastin, such as vitamin A, vitamin E and/or vitamin C,
and/or the dispense unit (1) for dispensing the photosensitizer containing agent being a tube, a box, a dosing unit, a gel band-aid or an applicator with a disposable syringe,
and/or the photosensitizer containing agent being fluorescent so as to determine the depth of penetration in the skin,
and/or the laser therapy system additionally comprising an alarm timer which indicates that the agent has sufficiently penetrated into the skin.

6. Laser therapy system according to one or more of the preceding claims, the first light source (2) comprising at least one UVA laser or one UVA lamp with the first wavelength in the range of 340-450 nm.

7. Laser therapy system according to one or more of the preceding claims, the second light source (3) comprising at least one IR laser with the second wavelength in a range of 800-2000 nm.

8. Laser therapy system according to one or more of the preceding claims 1-6, wherein the second light source (3) comprises a laser or a plurality of laser diodes with the second wavelength in a range from 450-550nm or 550-650nm or 650-800nm and is preferably selected therein such that it is absorbed as well as possible by hemoglobin and as little as possible by the other remaining skin tissue.

9. Laser therapy system according to one or more of the preceding claims, the first (2) and/or the second light source (3) comprising several individual light sources arranged at certain spaces so as to create several first and/or second light-beam bundles at the same time, so that these can be applied simultaneously at different positions of the treatment area.

10. Laser therapy system according to one or more of the preceding claims, the optical system (4) with its light-beam deflection and focusing optical system (5) comprising at least one lens (55), a concave lens, a cylindrical lens, a diffuser lens, a Fresnel lens, a liquid lens, a first lens system (53), a second lens system (54), a light conductor (56), a light-conducting fiber bundle (57), a light adapter head (58) or a combination thereof,
and/or
the cross-section in the focal point (52) being multiple times smaller than the first cross-section at light entry into the skin, the multiple being 3-1000,
and/or
the optical system (4) comprising a plurality of light-beam deflection and focusing optical systems (5) arranged therein which are adapted to create a corresponding plurality of focused light-beam bundles (51) and focal points (52) with respective light spots behind the adapter plate (7) and in the treatment area at the same time.

11. Laser therapy system according to one or more of the preceding claims, wherein the focal points (52) of the focused light-beam bundles (51) of the first light wavelength correspond to the focal points (52) of the focused light-beam bundles (51) of the second light wavelength, except for deviations due to different refractive indices at the different wavelengths, or wherein the focal points (52) of the focused light-beam bundles (51) of the first light wavelength are offset from the focal points (52) of the focused light-beam bundles (51) of the second light wavelength by a defined degree of offset.

12. Laser therapy system according to one or more of the preceding claims, the electromechanical adjusting device (6) comprising actuators which, for the purposes of light-beam deflection and/or shift in focal point, are connected to reflecting mirrors, semi-transmitting mirrors, to lens systems (53, 54), apertures, shifting elements, light conductors (56), optical filters (93) or combinations thereof so as to be able to position the respective focal points (52) in the treatment area deliberately in the x/y/z direction.

13. Laser therapy system according to one or more of the preceding claims, wherein the control unit (8) is adapted to create the light spots with the first and/or with the second wavelength behind the adapter plate (7) in the treatment area of the skin in an automated manner along a predefined or previously determined light-spot matrix, with the light spots being created either by at least two discrete simultaneous light spots or by a sequence of individual light spots,
and/or
wherein the laser therapy system keeps calibration parameters stored and/or wherein the laser therapy system can be calibrated by a calibration dummy before a treatment,
and/or
wherein the adapter plate (7) itself or a highly transparent upstream cooling plate has a cooling effect and is created to be applied directly on the skin above the treatment area located below it.

14. Laser therapy system according to one or more of the preceding claims, wherein the laser therapy system additionally comprises a dispense unit for a wound adhesive and/or for a band-aid.

15. Laser therapy system according to one or more of the preceding claims, wherein
a) the dispense unit (1) for dispensing the photosensitizer containing agent is adapted for treating the conjunctiva or the sclera of the eye,
b) the first light source (2) with the first light intensity and the first light pulse duration is adapted for treating the conjunctiva or the sclera of the eye,
c) the second light source (3) with the second light intensity and the second light pulse duration is adapted for treating the conjunctiva or the sclera of the eye,
d) the optical system (4) with the light-beam deviation and focusing optical system (5), the electromechanical adjusting device (6) and the adapter plate (7) is adapted for treating the conjunctiva or the sclera of the eye and
e) the control unit (8) is adapted for treating the conjunctiva or the sclera of the eye.

## Revendications

1. Système de thérapie au laser pour le traitement de la peau dans une zone de traitement en volume en profondeur dans la peau, comprenant :
a) une unité de délivrance (1) pour délivrer un produit contenant un agent photosensible à appliquer sur la peau;
b) une première source lumineuse (2) qui génère au moins un premier faisceau de rayons lumineux ayant une première longueur d'onde dans une plage UVA et dont la première intensité lumineuse et la première durée d'impulsion lumineuse sont contrôlables, la première longueur d'onde, la première intensité lumineuse et la première durée d'impulsion lumineuse étant établies et contrôlables de manière à former dans la peau des liaisons croisées de collagène et d'élastine à un point focal (52) du faisceau de rayons lumineux focalisé (51) en présence du produit contenant un agent photosensible;
c)une seconde source lumineuse (3) se présentant sous la forme d'une source de lumière laser et qui génère au moins un second faisceau de rayons lumineux ayant une seconde longueur d'onde et dont la seconde intensité lumineuse et la seconde durée d'impulsion lumineuse sont contrôlables, la seconde longueur d'onde, la seconde intensité lumineuse et la seconde durée d'impulsion lumineuse étant établies et contrôlables de manière à ce que, au point focal (52) du faisceau de rayons lumineux focalisé (51), les artérioles terminales deviennent perméables à des composants sanguins corpusculaires au centre au niveau d'une papille respective (23) du stratum papillaire de la zone de traitement sans blesser ou perforer alors un épiderme (21) se trouvant au-dessus;
d)un système optique (4)
qui est relié fixement aux première (2) et seconde (3) sources lumineuses et qui permet d'absorber au moins un premier et un second faisceaux de rayons lumineux;
comprenant une optique de déviation et de focalisation de rayons lumineux (5) qui présente un dispositif de réglage électromécanique (6) et une plaque d'adaptation transparente (7) faisant office d'orifice de sortie de lumière, l'optique de déviation et de focalisation de rayons lumineux (5) étant conçue pour être réglable et contrôlable au niveau électrique par le dispositif de réglage électromécanique (6) de manière à retransmettre, dévier et positionner en les focalisant avec une certaine distance focale les premier et second faisceaux de rayons lumineux absorbés de manière à ce que le faisceau de rayons lumineux respectif résultant (51) sorte derrière la plaque d'adaptation (7) avec une première surface de section transversale lumineuse et que son point focal respectif (52) soit défini très précisément derrière la plaque d'adaptation (7) par des coordonnées *x*/*y*/*z* et soit positionnable très précisément de manière contrôlée par rapport à la plaque d'adaptation (7) dans la zone de traitement en volume et
e)une unité de commande (8)
pour la commande du dispositif de réglage électromécanique (6) du système optique (4) pour la navigation du point focal et
pour la commande de la première (2) et de la seconde (3) source lumineuse, de sorte que la source lumineuse respective (2, 3) génère la première ou la seconde intensité lumineuse lors de la première ou seconde durée d'impulsion lumineuse pour créer ainsi dans les points focaux respectifs (52) des spots lumineux souhaités pour des réactions respectives comme la liaison croisée de collagène et d'élastine ou une perméabilité des artérioles terminales pour les composants sanguins corpusculaires.

2. Système de thérapie au laser selon la revendication 1, ce système de thérapie au laser comprenant en outre une unité d'imagerie dotée d'un système de caméra (9) et d'un moniteur (10), une unité de caméra (91) du système de caméra (9) étant raccordée au système optique (4) et présentant un champ de détection (94) qui recouvre grâce à un parcours optique du système optique (4) la zone de traitement située derrière la plaque d'adaptation (7),
une image s'affichant sur le moniteur (10) de la zone de traitement avec les coordonnées *x*/*y*/*z* peut être ajustée pour concorder avec les coordonnées *x*/*y*/*z* du système optique (4) de l'unité de commande (8) et en fin de compte avec les points focaux (52) de manière à ce que le spot lumineux dans la zone de traitement puisse être généré précisément à un point affiché dans l'image
et/ou
l'unité de commande (8) prenant en compte, lors du calcul des coordonnées *x*/*y*/*z* des points focaux (52) dans la zone de traitement située derrière la plaque d'adaptation (7), de la réfraction de lumière de la peau et d'un éventuel film supplémentaire ou d'un pansement situé entre la plaque d'adaptation (7) et la peau.

3. Système de thérapie au laser selon la revendication 2, dans lequel le système de caméra (9) intègre une tomographie à cohérence optique (TCO), une microscopie à fluorescence ou une tomographie à fluorescence et permet une représentation tridimensionnelle de la zone de traitement de la peau et permet de rendre visibles à partir de là les fibres de collagène (25, 26), l'élastine, les racines des cheveux, les papilles (23), les artérioles, les artérioles terminales, le tissu cutané traité, le tissu cutané pas encore traité ou une combinaison de ceux-ci;
ou
le système de caméra (9) repose sur une mesure de réflexion lumineuse ou sur une mesure d'extinction au moyen de l'unité de caméra (91), sachant qu'on peut sélectionner pour une prise de cliché un éclairage ayant une première longue d'onde d'éclairage qui est absorbé ou reflété particulièrement fortement dans les zones artérielles de la peau de manière à détecter de manière particulièrement contrastée une zone irriguée par les artères et ainsi les artérioles et artérioles terminales et à les représenter sur l'image;
ou
le système de caméra (9) prévoit pour la mesure de réflexion lumineuse au moyen d'une unité de caméra (91) une source d'éclairage (92) ayant au moins deux longueurs d'ondes d'éclairage différentes, sachant que, dans une première étape, au moins deux images de caméra peuvent être générées aux longueurs d'ondes d'éclairage différentes et enregistrées, la longueur d'onde d'éclairage respective pouvant être adaptée aux composants de la peau à détecter principalement et à contraster et une des longueurs d'ondes d'éclairage étant égale ou inégale à la première ou seconde longueur d'onde d'éclairage et, dans une seconde étape, l'image de la zone de traitement pouvant être générée et représentée par un traitement d'image à partir des au moins deux images de caméra enregistrées, le traitement d'image totalisant, soustrayant l'une de l'autre, mettant en logarithme puis soustrayant l'une de l'autre les deux images de caméra, effectuant ensuite un relèvement des bords ou exploitant les images de caméra avec une sélection de couleurs RJB.

4. Système de thérapie au laser selon la revendication 2 ou 3, dans lequel l'image affiche la zone de traitement derrière la plaque d'adaptation (7) et la zone papillaire comportant les papilles (23) peut être détectée à l'aide d'une unité de processeur à partir d'une détection de modèle et les positions à traiter avec des spots lumineux peuvent être définies en donnant ensemble la matrice des spots lumineux au moyen de spots lumineux dans la première et/ou seconde longueur d'onde;
et/ou
un des processeurs du système de thérapie au laser, grâce à une seconde détection continue de modèle dans l'image de la zone de traitement située derrière la plaque d'adaptation (7), détecte un éventuel décalage de la plaque d'adaptation (7) sur la peau et détecte en outre quelle partie de la zone de traitement a déjà été traitée et quelle partie non traitée de la zone de traitement reste à traiter, la partie traitée et la partie non traitée de la zone de traitement pouvant être représentées en couleurs différentes sur le moniteur (10) et une génération automatisée correspondante de spots lumineux pouvant être effectuée.

5. Système de thérapie au laser selon une ou plusieurs des revendications précédentes, dans lequel le produit contenant de l'agent photosensibilisant est une crème ou un gel ayant un effet de pénétration dans la peau et de favorisation de la formation de collagène et d'élastine et qui contient des substances comme la riboflavine (vitamine B2) et des coenzymes pour une synthèse du collagène et de l'élastine, comme de la vitamine A, de la vitamine E et/ou de la vitamine C,
et/ou l'unité de délivrance (1) servant à délivrer le produit contenant de l'agent photosensibilisant est un tube, une boîte, une unité de dosage, un emplâtre au gel ou un applicateur doté d'une seringue à usage unique,
et/ou le produit contenant de l'agent photosensibilisant est fluorescent afin de définir ainsi la profondeur de pénétration dans la peau,
et/ou le système de thérapie au laser comprend en outre un temporisateur à alarme qui indique que le produit a suffisamment pénétré dans la peau.

6. Système de thérapie au laser selon une ou plusieurs des revendications précédentes, dans lequel la première source lumineuse (2) comprend au moins un laser UVA ou une lampe UVA ayant la première longueur d'ondes de l'ordre de 340 à 450 nm.

7. Système de thérapie au laser selon une ou plusieurs des revendications précédentes, dans lequel la seconde source lumineuse (3) comprend au moins un laser IR ayant la seconde longueur d'ondes de l'ordre de 800 à 2000 nm.

8. Système de thérapie au laser selon une ou plusieurs des revendications précédentes 1-6, dans lequel la seconde source lumineuse (3) comprend au moins un laser ou une multitude de diodes laser ayant la seconde longueur d'ondes de l'ordre de 450 à 550 ou 550 à 650 nm ou de 650 à 800 nm et est choisie dedans de préférence de manière à ce qu'elle soit absorbée aussi bien que possible par l'hémoglobine et aussi peu que possible par un tissu restant de la peau.

9. Système de thérapie au laser selon une ou plusieurs des revendications précédentes, dans lequel la première (2) et/ou seconde source lumineuse (3) comprend plusieurs sources lumineuses distinctes qui sont disposées avec certains écarts pour générer en même temps plusieurs premiers et/ou seconds faisceaux de rayons lumineux de manière à ce que ceux-ci puissent être appliqués en même temps à des points différents de la zone de traitement.

10. Système de thérapie au laser selon une ou plusieurs des revendications précédentes, dans lequel le système optique (4) avec son optique de déviation et de focalisation de rayons lumineux (5) comprend au moins une lentille (55), une lentille concave, une lentille cylindrique, une lentille de dispersion, une lentille Fresnel, une lentille à liquide, un premier système de lentille (53), un second système de lentille (54), une fibre optique (56), un faisceau de fibres optiques (57), une tête d'adaptation de lumière (58) ou une combinaison de ceux-ci;
et/ou
la surface de section transversale est au point focal (52) de multiples fois inférieure à la première surface de section transversale à l'entrée de la lumière dans la peau, ce multiple étant de 3 à 1000;
et/ou
le système optique (4) comprend une pluralité d'optiques de déviation et de focalisation de rayons lumineux (5) disposées dedans et qui sont conçues pour générer simultanément une multitude correspondante de faisceaux de rayons lumineux focalisés (51) et générer des points focaux (52) avec des spots lumineux correspondants derrière la plaque d'adaptation (7) et dans la zone de traitement.

11. Système de thérapie au laser selon une ou plusieurs des revendications précédentes, dans lequel les points focaux (52) des faisceaux de rayons lumineux focalisés (51) de première longueur d'onde d'éclairage coïncident avec les points focaux (52) des faisceaux de rayons lumineux focalisés (51) de seconde longueur d'onde d'éclairage, hormis les écarts dus à différents indices de réfraction dans les différentes longueurs d'onde ou dans lequel les points focaux (52) des faisceaux de rayons lumineux focalisés (51) de première longueur d'onde d'éclairage sont décalés des points focaux (52) des faisceaux de rayons lumineux focalisés (51) de seconde longueur d'onde d'éclairage à raison d'un décalage défini.

12. Système de thérapie au laser selon une ou plusieurs des revendications précédentes, dans lequel le dispositif de réglage électromécanique (6) comprend des actionneurs qui sont raccordés en vue d'une déviation des rayons lumineux et/ou d'un réglage du point focal à l'aide de miroirs déviateurs, de miroirs semi-transparents, de systèmes de lentilles (53, 54), de caches, d'éléments coulissants, de fibres optiques (56), de filtres optiques (93) ou de combinaisons de ceux-ci afin de pouvoir positionner les points focaux respectifs (52) dans la zone de traitement délibérément dans le sens *x*/*y*/*z.*

13. Système de thérapie au laser selon une ou plusieurs des revendications précédentes, dans lequel l'unité de commande (8) est conçue pour générer de manière automatisée les spots lumineux ayant la première et/ou seconde longueur d'onde derrière la plaque d'adaptation (7) dans la zone de traitement de la peau le long de matrices de spots lumineux prédéfinies ou dans des matrices de spots lumineux définies au préalable, les spots lumineux étant générés soit par au moins deux spots lumineux discrets simultanés, soit par une succession séquentielle de spots lumineux distincts;
et/ou
le système de thérapie au laser garde des paramètres d'étalonnage enregistrés et/ou le système de thérapie au laser peut être étalonné à l'aide d'une variable d'essai d'étalonnage avant un traitement;
et/ou
la plaque d'adaptation (7) elle-même ou une plaque réfrigérante transparente installée en amont a un effet réfrigérant et est conçue pour être appliquée directement sur la peau sur la zone de traitement sous-jacente.

14. Système de thérapie au laser selon une ou plusieurs des revendications précédentes, ce système de thérapie au laser comprenant en outre une unité de délivrance de sparadrap et/ou de pansement.

15. Système de thérapie au laser selon une ou plusieurs des revendications précédentes, dans lequel
a) l'unité de délivrance (1) assurant la délivrance du produit contenant de l'agent photosensibilisant est adaptée pour traiter la conjonctive ou la sclérotique de l'oeil;
b) la première source lumineuse (2) ayant la première intensité lumineuse et la première durée d'impulsion lumineuse est adaptée pour traiter la conjonctive ou la sclérotique de l'oeil;
c) la seconde source lumineuse (3) ayant la seconde intensité lumineuse et la seconde durée d'impulsion lumineuse est adaptée pour traiter la conjonctive ou la sclérotique de l'oeil;
d) le système optique (4) doté de l'optique de déviation et focalisation de rayons lumineux (5) du dispositif de réglage électromécanique (6) et de la plaque d'adaptation (7) est adapté pour traiter la conjonctive ou la sclérotique de l'oeil ; et
e) l'unité de commande (8) est adaptée pour traiter la conjonctive ou la sclérotique de l'oeil.
